# EUROPEAN PATENT APPLICATION

(11) **EP 1 498 137 A1**
(43) Date of publication of application: **19.01.2005**
(21) Application number: 03719232.5
(22) Date of filing: 25.04.2003
(51) Int. Cl.: A61K 39/395, A61K 31/7088, A61K 48/00, A61P 11/00, A61P 35/00

(54) **REMEDY FOR LUNG CANCER**

(30) Priority: 25.04.2002 JP 2002124743
(71) Applicant: CHUGAI SEIYAKU KABUSHIKI KAISHA, Tokyo, 115-8543 (JP)
(72) Inventor: NEZU, Jun-ichi c/o Chugai Seiyaku Kabushiki Kaisha, Niihari-gun,Ibaraki 300 (JP)
(74) Representative: Soames, Candida Jane
(86) International application number: PCT/JP2003/005399
(87) International publication number: WO 2003/090779

(57) **Abstract**

The present invention provides a pharmaceutical composition for treating lung carcinoma and a diagnostic agent for lung carcinoma. More specifically, the present invention provides a pharmaceutical composition for treating lung carcinoma and a diagnostic agent for lung carcinoma, which comprise an antibody capable of binding to the β-chain of an interleukin-20 receptor, and a method for detecting lung carcinoma, which comprises detecting the expression of the β-chain of an interleukin-20 receptor in a sample obtained from a mammal suspected to have lung carcinoma.

## Description

### Technical Field

The present invention relates to a pharmaceutical composition for treating lung carcinoma, and a diagnostic agent and a detection method for lung carcinoma.

### Background Art

In Japan, lung carcinoma is the first leading cause of cancer death regarding men, and the number of patients has been continuously increasing. Regarding women, gastric carcinoma still remains as the first leading cause of cancer death. However, lung carcinoma is the second leading cause of cancer death, and the gap between the number of cancer death caused by gastric carcinoma and that caused by lung carcinoma has been decreasing year by year. Thus, as in the case of men, lung carcinoma tends to increase in women. Lung carcinoma is pathologically classified into small cell lung carcinoma (SCLC), adenocarcinoma, squamous cell lung carcinoma, and large cell lung carcinoma. From a therapeutic viewpoint, the last three types of carcinomas are often dealt collectively as non small cell lung carcinoma (NSCLC). It is said that non small cell lung carcinoma makes up approximately 85% of all lung carcinomas (Table 1).

**Table 1**

| Classification of Lung Carcinoma | | |
|---|---|---|
| 1. | Small Cell Lung Carcinoma (SCLC) | Approximately 15% |
| 2. | Non Small Cell Lung Carcinoma (NSCLC) | Approximately 85% |
| | • Adenocarcinoma | |
| | • Squamous Cell Lung Carcinoma | |
| | • Large Cell Lung Carcinoma | |

When non small cell lung carcinoma is compared with small cell lung carcinoma which highly responds treatments such as chemotherapy, non small cell lung carcinoma has extremely low chemosensitivity. In spite of the recent development of radiotherapy or combination chemotherapy mainly utilizing CDDP, these therapies provide extremely low life-prolonging effects to patients with non small cell lung carcinoma. Thus, it cannot be said that the results of the therapies are sufficient. In other words, non small cell lung carcinoma is one of cancers with extremely poor prognosis. Therefore, it is strongly desired that an innovative therapeutic agent be developed.

Many attempts have previously been made to explore cancer-associated genes, based on the difference in the expression level of the genes. Most of these approaches have been simple comparisons between the expression level of genes in cancer tissues and that in the corresponding normal tissues. It is considered that, in such methods, many genes are simultaneously found based on common basic cell growth mechanisms. Such genes are highly likely to be necessary not only for the growth of carcinoma cells but also for the growth of normal cells. Thus, it is predicted that an agent targeting such genes affects even normal cells with a high growth level, such as hematopoietic cells or epithelial cells in the small intestine. Such an agent basically has no advantages when compared with the existing chemotherapeutic agents such as antimetabolites.

Accordingly, it has been required to specify a gene, which is more specifically involved in growth or malignant alteration of lung carcinoma cells than in normal cells with a high growth level. In addition, it is necessary to develop a therapeutic agent effective for treating lung carcinoma by using the thus specified gene, wherein the therapeutic agent prevents the development of lung carcinoma, and especially the development of non small cell lung carcinoma, but does not affect at all the growth of normal cells. Further, utilizing the specificity of the above-described gene, it is also desired to develop a diagnostic agent capable of detecting lung carcinoma at the early stage.

On the other hand, concerning a factor associated with cell growth, it has been disclosed that interleukin-20 (hereinafter referred to as IL-20) is an important factor for the growth of keratinocytes on the skin, that IL-20 receptors are excessively expressed in patients with human psoriasis, and that a substance blocking signals via the IL-20 receptor can be an antipsoriatic agent (Cell. 2001 Jan 12; 104 (1): 9-19). The aforementioned report, however, has no descriptions regarding findings about IL-20 or an IL-20 receptor associated with the growth of lung carcinoma cells, or regarding the action of signals via the IL-20 receptor on the growth of carcinoma cells.

### Disclosure of the Invention

It is an object of the present invention to provide a pharmaceutical composition for treating lung carcinoma, and a diagnostic agent and a detection method for lung carcinoma.

The present inventor has conducted intensive studies to achieve the above object. The present invention has explored a lung carcinoma target gene by a subtraction method, and as a result, the inventor has found that an interleukin-20 receptor β-chain gene is increasingly expressed in lung carcinoma cells at a high level and at a high frequency, thereby completing the present invention.

That is to say, the present invention provides the following (1) to (11).
(1) A pharmaceutical composition for treating lung carcinoma, which comprises, as an active ingredient, an antibody binding to the β-chain of an interleukin-20 receptor.
(2) The pharmaceutical composition according to (1) above, wherein the antibody is a monoclonal antibody or polyclonal antibody.
(3) The pharmaceutical composition according to (1) or (2) above, wherein the antibody is a human antibody, humanized antibody, or chimeric antibody.
(4) A pharmaceutical composition for treating lung carcinoma comprising, an active ingredient, a polynucleotide which has a nucleotide sequence complementary to the nucleotide sequence of a polynucleotide encoding the β-chain of an interleukin-20 receptor and which is an antisense polynucleotide capable of suppressing the expression of the polynucleotide.
(5) A diagnostic agent for lung carcinoma, which comprises an antibody capable of binding to the β-chain of an interleukin-20 receptor.
(6) The diagnostic agent according to (5) above, wherein the antibody is a monoclonal antibody or polyclonal antibody.
(7) The diagnostic agent according to (5) or (6) above, wherein the antibody is a human antibody, humanized antibody, or chimeric antibody.
(8) A method for detecting lung carcinoma in mammals using the diagnostic agent according to any of (5) to (7) above.
(9) A method for detecting lung carcinoma, which comprises detecting the expression of the β-chain of an interleukin-20 receptor in a sample obtained from a mammal suspected to have lung carcinoma.
(10) The method according to (9) above, wherein an antibody capable of binding to the β-chain of an interleukin-20 receptor is used to detect the expression.
(11) The method according to (10) above, wherein the PCR method is used to detect the expression.

This specification includes part or all of the contents as disclosed in the specification and/or drawings of Japanese Patent Application No. 2002-124743, which is a priority document of the present application.

### Brief Description of the Drawings

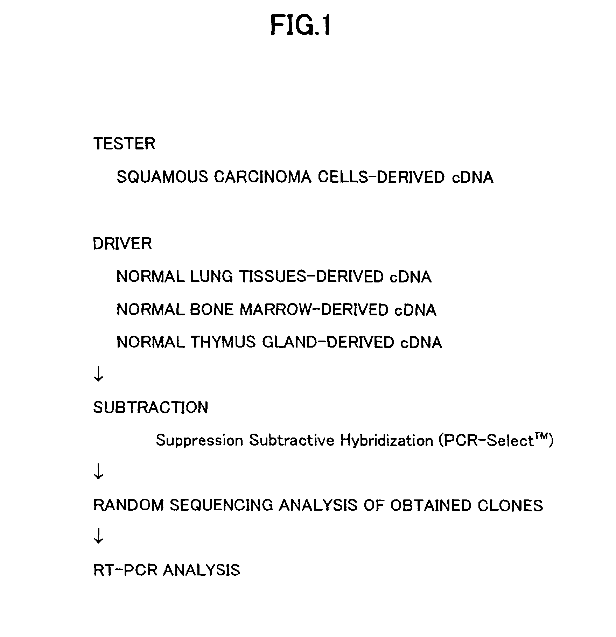

Figure 1 shows the scheme of a subtraction method in the present invention.

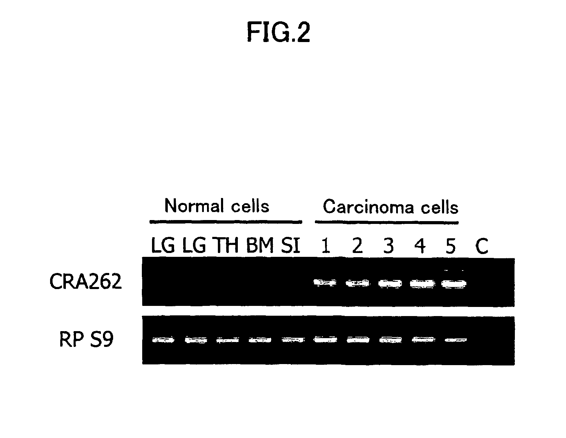

Figure 2 shows the results of electrophoresis obtained by RT-PCR analysis. In the figure, LG represents a normal lung, TH represents a normal thymus gland, BM represents a normal bone marrow, SI represents a normal small intestine, and C represents a control.

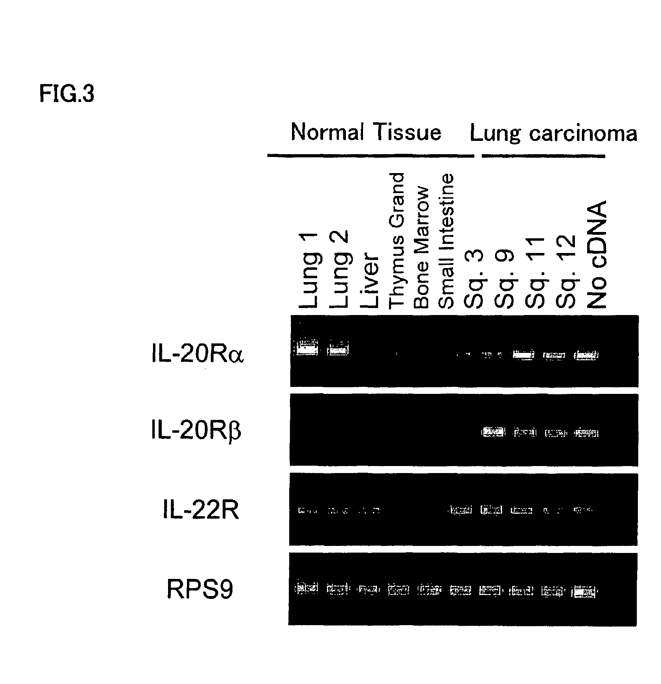

Figure 3 shows the results of RT-PCR analysis of expression of human genes (IL-20Rα, IL-20Rβ, IL-22R, and RP S9) in normal human tissues (lung, liver, thymus gland, bone marrow, and small intestine) and in non small lung carcinoma clinical samples (Sq. 3, Sq. 9, Sq. 11, and Sq. 12). The term "no cDNA" means the case where template DNA was not used.

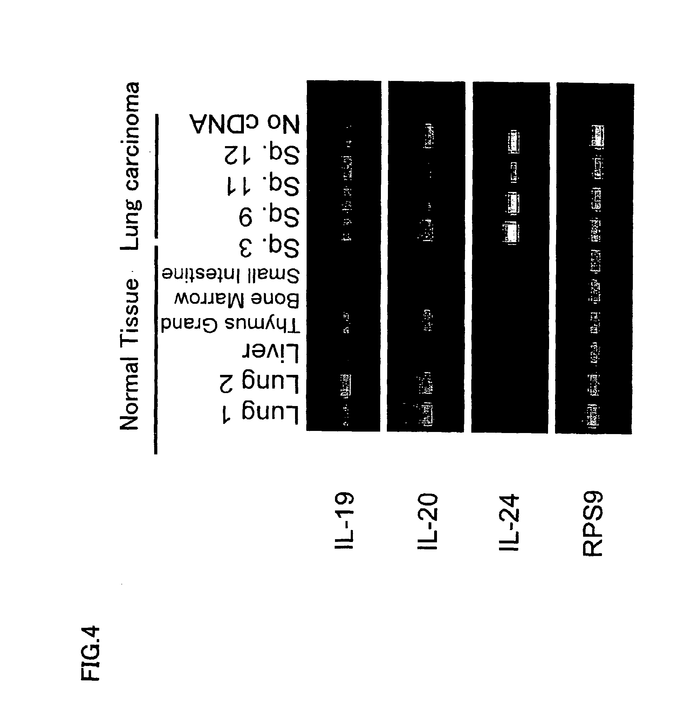

Figure 4 shows the results of RT-PCR analysis of expression of human genes (IL-19, IL-20, IL-24, and RP S9) in normal human tissues (lung, liver, thymus gland, bone marrow, and small intestine) and in non small lung carcinoma clinical samples (Sq. 3, Sq. 9, Sq. 11, and Sq. 12). The term "no cDNA" means the case where template DNA was not used.

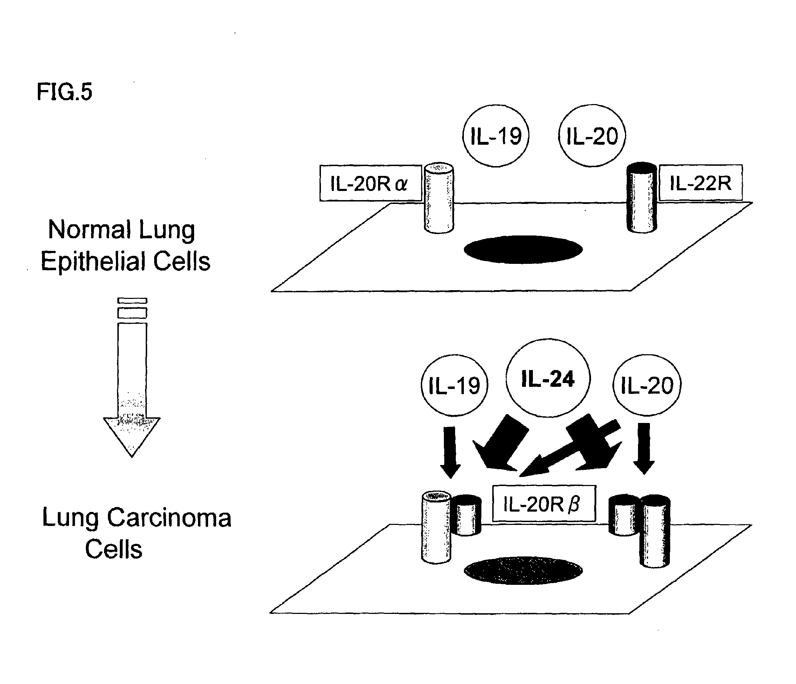

Figure 5 schematically shows the involvement of signals via IL-20Rβ in canceration of lung epithelial cells. A circle means each cytokine, and a cylinder means a receptor of the cytokine. An arrow means a receptor to which each cytokine can bind. The upper diagram shows the state of signals via IL-20Rβ in normal lung epithelial cells, and the lower diagram shows the state of signals via IL-20Rβ in cancerous lung epithelial cells. The expression of IL-20Rβ and IL-24 is increased by canceration, so that signals caused by these genes can efficiently be transduced into cells.

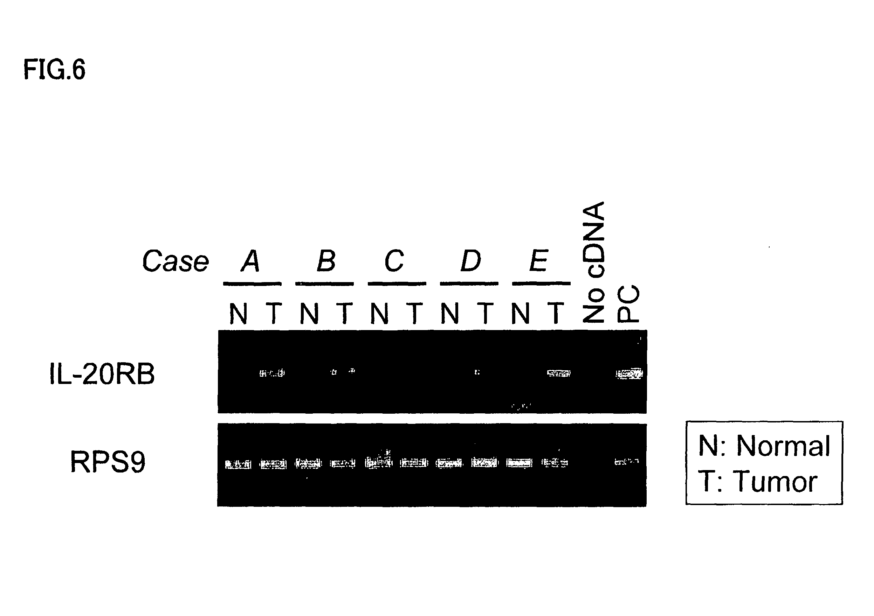

Figure 6 shows the results of RT-PCR analysis of expression of human IL-20Rβ gene (arrow) and human RP S9 gene in samples derived from patients with lung carcinoma. N represents a non-tumor portion-derived sample, and T represents a tumor portion-derived sample. The term "no cDNA" means a negative control where no template DNA is used, and the term "PC" means a positive control where lung carcinoma-derived cDNA is used wherein IL-20Rβ is highly expressed.

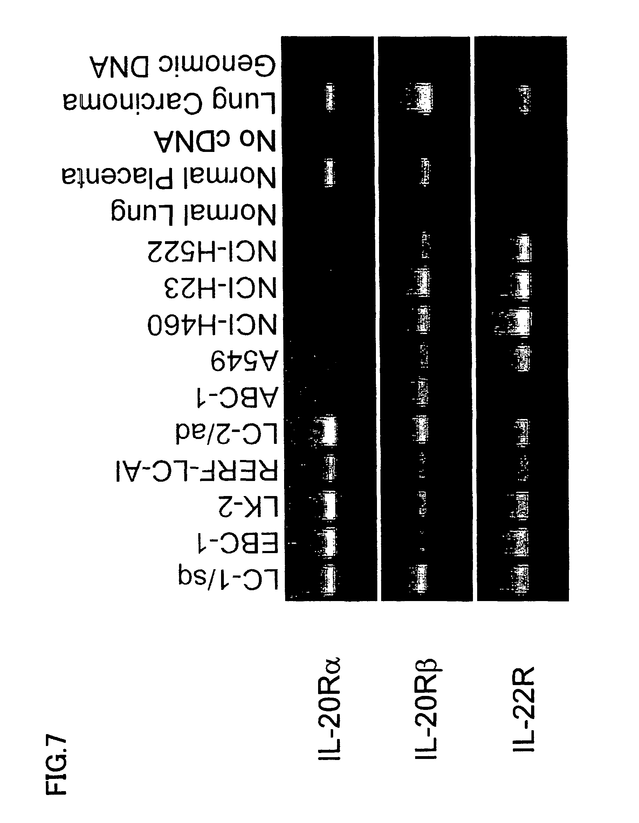

Figure 7 shows the result of RT-PCR analysis of expression of human genes (IL-20Rα, IL-20Rβ, and IL-22R) in various types of human lung carcinoma cell lines (LC-1/sq, EBC-1, LK-2, RERF-LC-AI, LC-2/ad, ABC-1, A549, NCI-H460, NCI-H23, and NCI-H522). The term "no cDNA" means the case where no template DNA is used, and the term "genomic DNA" means the case where human chromosomal DNA is used as a template.

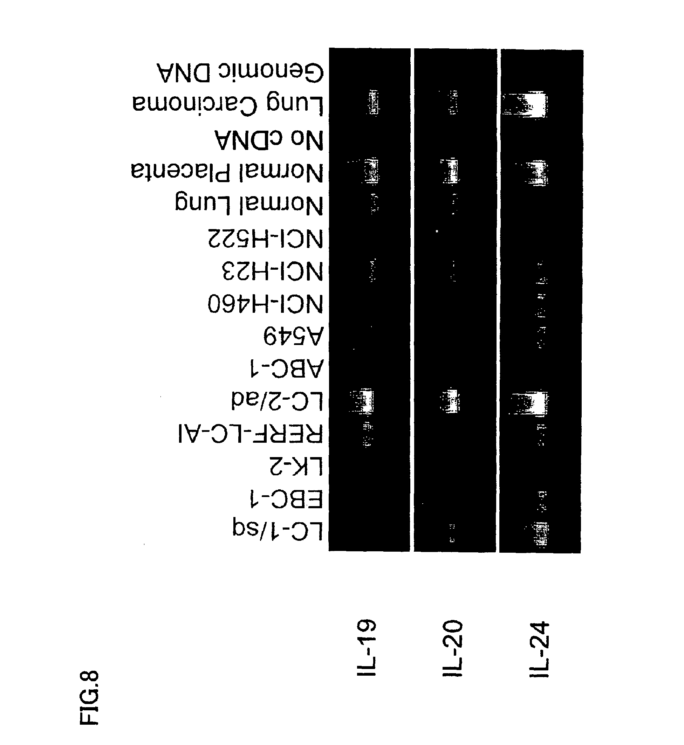

Figure 8 shows the results of RT-PCR analysis of expression of human genes (IL-19, IL-20, and IL-24) in various types of human lung carcinoma cell lines (LC-1/sq, EBC-1, LK-2, RERF-LC-AI, LC-2/ad, ABC-1, A549, NCI-H460, NCI-H23, and NCI-H522). The term "no cDNA" means the case where no template DNA is used, and the term "genomic DNA" means the case where human chromosomal DNA is used as a template.

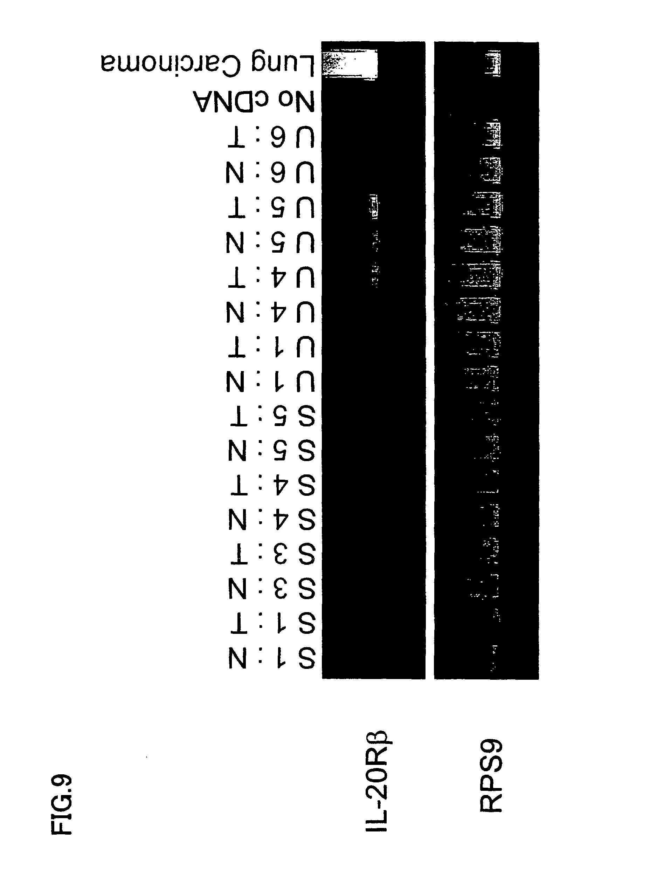

Figure 9 shows the results of RT-PCR analysis of expression of human genes (IL-20Rβ and RP S9) in samples derived from patients with gastric carcinoma (S1, S3, S4, and S5) and in samples derived from patients with uterine carcinoma (U1, U4, U5, and U6). In the samples, N represents a non-tumor portion-derived sample, and T represents a tumor portion-derived sample. The term "no cDNA" means the case where no template DNA is used.

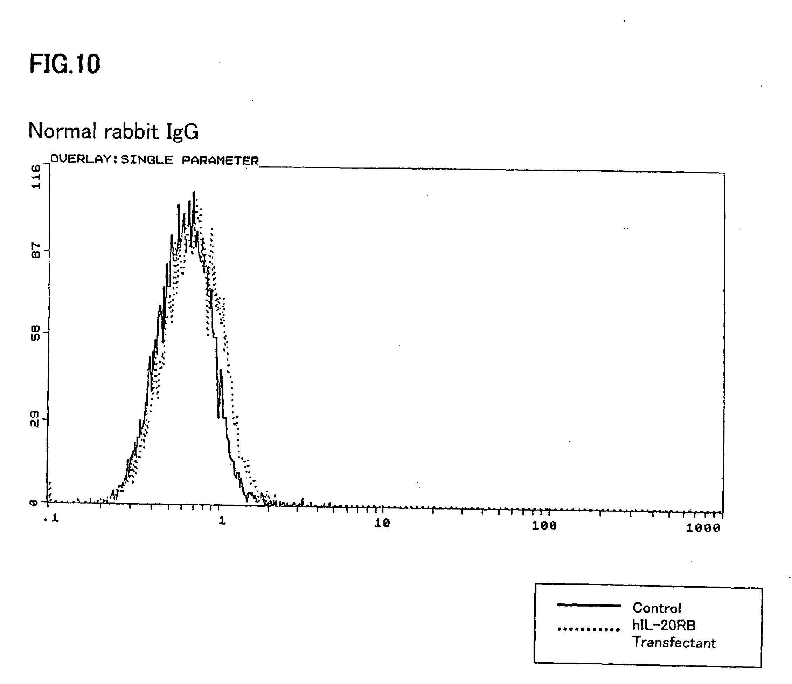

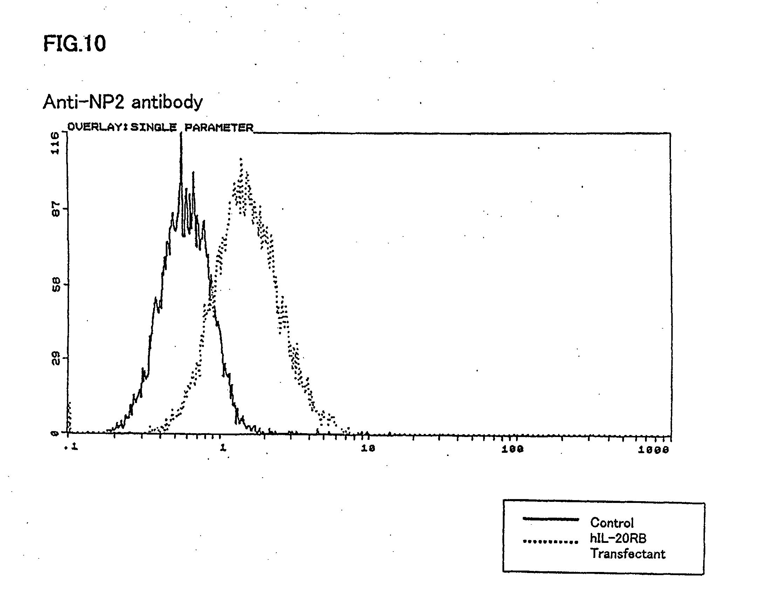

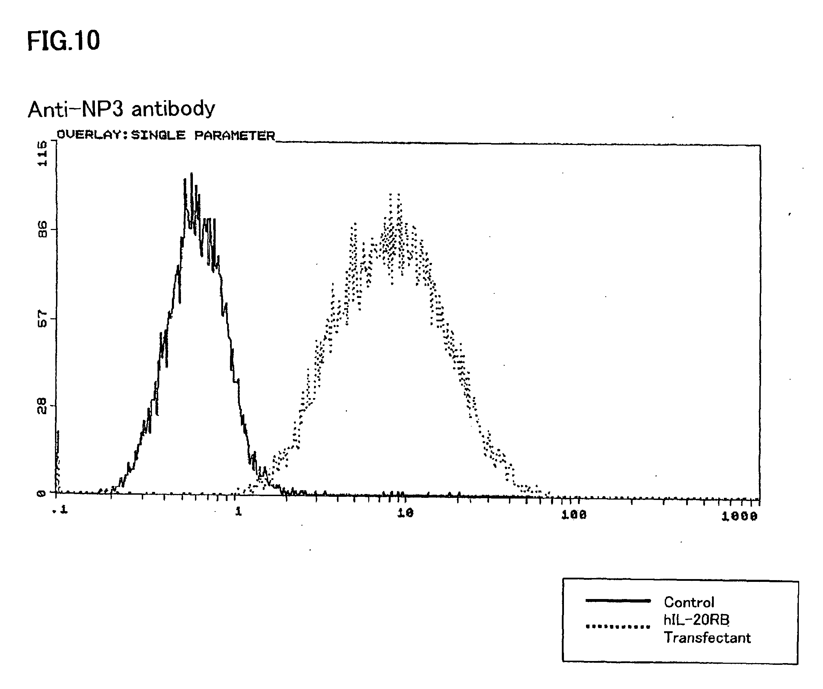

Figure 10 shows the results of FACS analysis using an anti-IL-20Rβ rabbit polyclonal antibody. The term "hIL-20RB transfectant" means a cell line wherein human IL-20Rβ is forced to be expressed (cell line 3), and the term "control" means cells obtained by allowing only a vector to express in the same parent cells. Cell line 3 is specifically stained with an anti-NP2 antibody and an anti-NP3 antibody, and it is therefore found that these antibodies specifically react with human IL-20Rβ. The term "normal rabbit IgG" means a negative control where IgG purified from normal rabbits is used as a primary antibody.

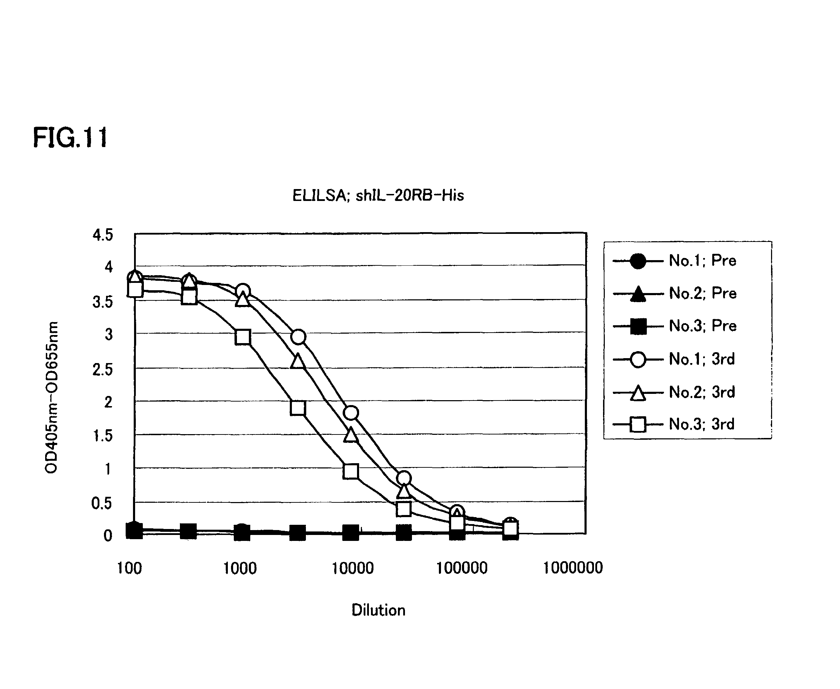

Figure 11 shows the results of ELISA analysis of the serum from mice (No. 1, No. 2, and No. 3) immunized with a soluble human IL-20Rβ-FLAG protein. The term "Pre" means the serum before immunization, and the term "3rd" means the serum after three immunizations. A soluble human IL-20Rβ-His protein is used as an antigen for ELISA. The horizontal axis ("dilution") indicates the dilution ratio of the serum.

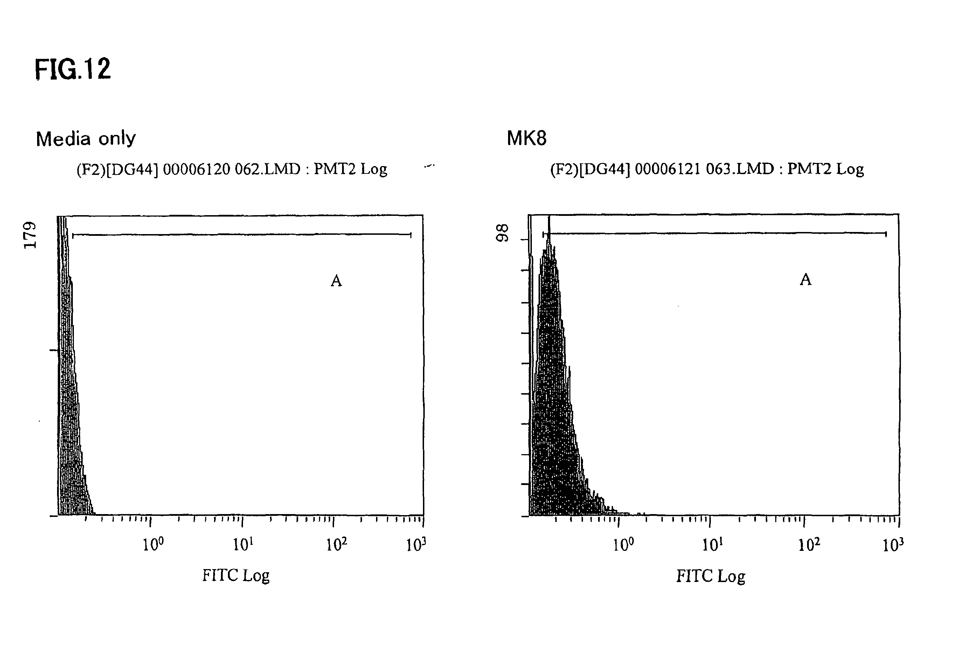

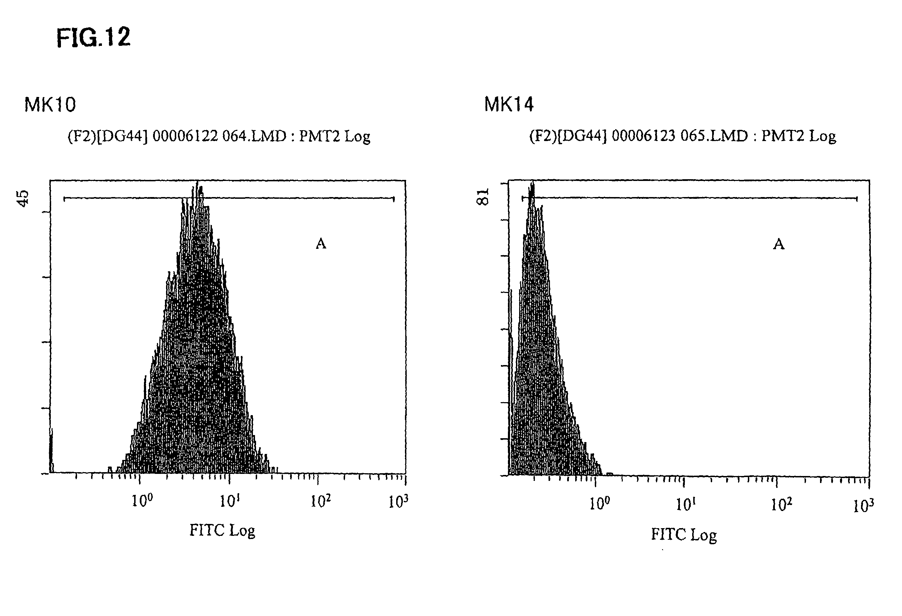

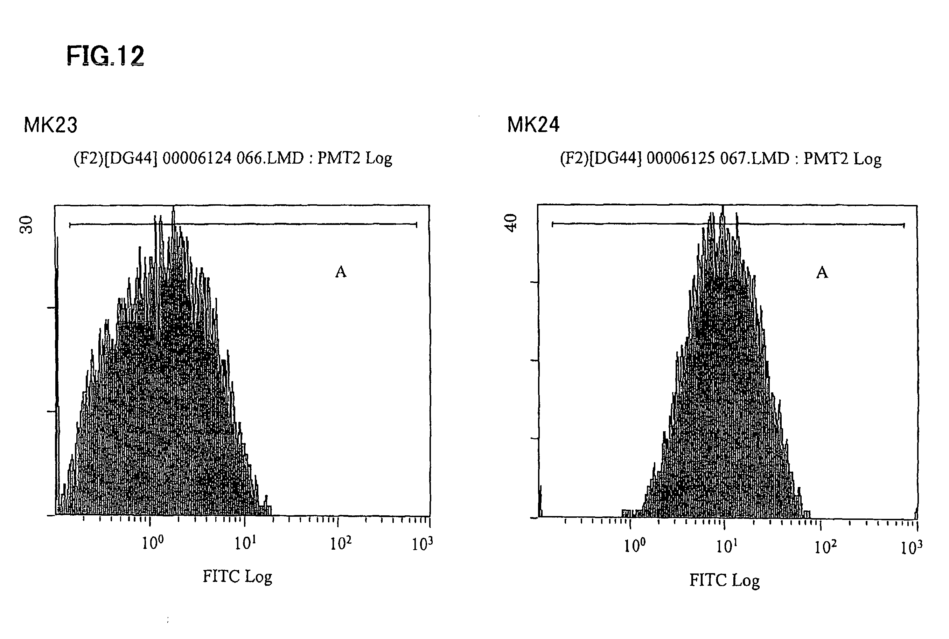

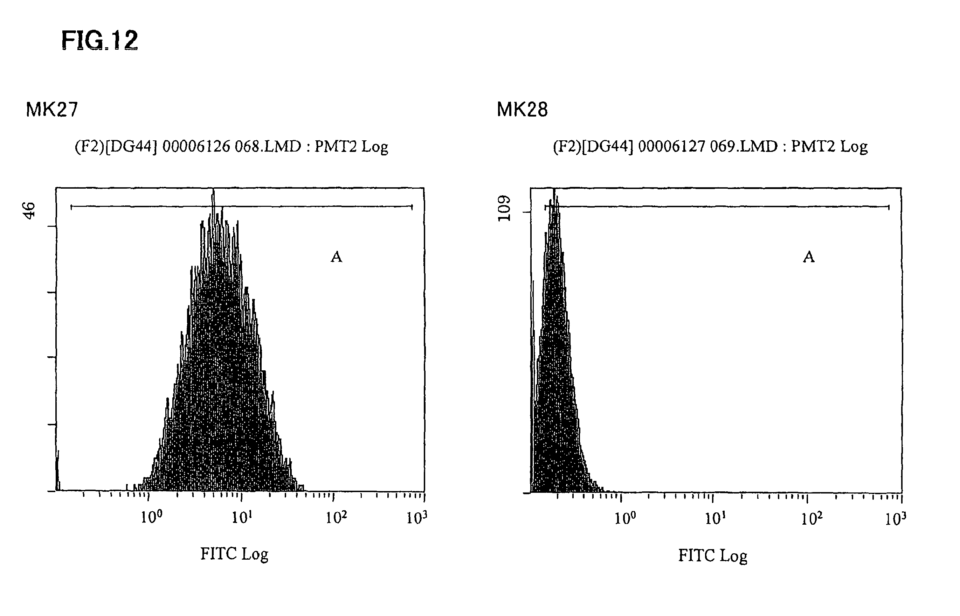

Figure 12 shows the results of FACS analysis using an anti-IL-20Rβ monoclonal antibody (MK clone). A cell line (cell line 3) wherein human IL-20Rβ is forced to be expressed is specifically stained, and it is therefore found that these antibodies specifically react with human IL-20Rβ. The term "medium only" means a negative control wherein only a hybridoma medium is used as a primary antibody.

### Best Mode for Carrying Out the Invention

The present invention will be described further in detail below.

The term "treatment" used in the present specification includes administration of the pharmaceutical composition of the present invention to a subject to cause lung carcinoma to disappear, to suppress further progression of lung carcinoma, and to alleviate symptoms caused by lung carcinoma.

An antibody used for the pharmaceutical composition for treating lung carcinoma and the diagnostic agent for lung carcinoma of the present invention is not particularly limited, but any antibody may be used as long as it binds to the β-chain of an interleukin-20 receptor. It is preferably a monoclonal antibody. In addition, a monoclonal antibody having cytotoxicity such as antibody-dependent cellular cytotoxicity (ADCC) or complement-dependent cytotoxicity (CDC), or a monoclonal antibody capable of blocking signals that can be transduced into cells via the β-chain of an interleukin-20 receptor, may also be used.

Moreover, an antibody capable of suppressing the growth of cells expressing the β-chain of an interleukin-20 receptor by binding a radioactive isotope (RI) or toxin thereto according to known methods may also be included in the antibody of the present invention.

Furthermore, antibody fragments such as Fab or F(ab)2 and peptides such as a single-strand Fv, which are prepared based on the above antibody, may also be included in the antibody of the present invention.

### 1. Exploration of target gene for lung carcinoma cell by subtraction method

Many attempts have previously been made to explore cancer-associated genes, based on the difference in the expression level of the genes. Most of them are simple approaches wherein the expression level of genes in cancer tissues is compared with that in the corresponding normal tissues. In such methods, however, it is predicted that many genes are found based on common basic cell growth mechanisms. Moreover, such genes are highly likely to be necessary not only for the growth of carcinoma cells but also for the growth of normal cells. Thus, it is predicted that an agent that targets genes found by the conventional subtraction method affects even normal cells with a high growth level, such as hematopoietic cells or epithelial cells in the small intestine. Such an agent basically has no advantages when compared with the existing chemotherapeutic agents such as antimetabolites.

Hence, when genes expressing in normal lung tissues are subtracted from gene expressing in lung carcinoma tissues to find genes associated specifically with growth or malignant alteration of lung carcinoma cells, the present inventors simultaneously subtract genes expressing in hematopoietic cells. More specifically, using polyA⁺ RNA derived from squamous cell lung carcinoma as a tester (subtracted side), and using a mixture obtained by mixing polyA⁺ RNA derived from normal lung and polyA⁺ RNA derived from normal bone marrow and normal thymus gland, which are tissues containing large quantities of hematopoietic cells, as a driver (subtracting side), subtraction was carried out by suppression subtractive hybridization method (Proc Natl Acad Sci USA. 1996 Jun 11; 93 (12): 6025-30) (see, Figure 1).

A gene obtained by such a subtraction method is selectively expressed in lung carcinoma cells, but is not expressed in proliferative normal hematopoietic cells. Accordingly, it is considered that this gene is not associated with a general cell growth mechanism. In fact, as a result of analyzing a clone subtracted by this method, a gene CRA262 was found, which was increasingly expressed in non small cell carcinoma cells (see, Examples 1 to 3 and Figure 2).

It was confirmed that this gene was expressed in normal lung tissues in an extremely small amount, but that it was increasingly expressed in all the non small cell lung carcinoma samples obtained from 5 different patients with lung carcinoma. In addition, this gene was expressed in only a little amount in the bone marrow and thymus gland containing proliferative hematopoietic cells, and in the small intestine containing large quantities of proliferative epithelial cells. From these results, it is considered that this gene is plays a certain important role specific for growth or malignant alteration of lung carcinoma cells, and therefore that it can be an ideal target of a lung carcinoma therapeutic agent, which inhibits the progression of lung carcinoma but does not affect the growth of normal cells. Thus, this gene was used as a material for the following studies.

Database (GenSeq) regarding nucleic acids and amino acids in patent documents or the like was searched for this gene. As a result, it was confirmed that this gene is an IL-20 receptor β chain (hereinafter referred to as IL-20Rβ) gene. This IL-20Rβ gene had been discovered by several groups (International Patent Publication WO 99/46379, WO 00/53758, WO 00/39161, WO 99/62934, etc.). The nucleotide sequence and amino acid sequence of human IL-20Rβ cDNA are shown in SEQ ID Nos. 15 and 16, respectively.

As stated above, it was clarified that the IL-20Rβ gene is increasingly expressed at a high level and at a high frequency in lung carcinoma cells, and in particular in non small cell lung carcinoma cells, and that the gene is deeply associated with the growth of lung carcinoma cells. Accordingly, these findings provide a pharmaceutical composition for treating lung carcinoma and a diagnostic agent for lung carcinoma, using an antibody binding to IL-20Rβ or an antisense polynucleotide to the sequence (polynucleotide) of a gene encoding IL-20Rβ, which will be described below.

### 2. Antibody capable of binding to IL-20Rβ

### (1) Antibody

The antibody used in the present invention are not particularly limited to any particular origin, type (polyclonal antibody or monoclonal antibody), and form, as long as it is an antibody capable of binding to IL-20Rβ (hereinafter referred to as an anti-IL-20Rβ antibody).

For example, an antibody is not particularly limited, as long as it binds to an antigen, IL-20Rβ. Examples of an antibody appropriately used herein may include a mouse antibody, a rat antibody, a rabbit antibody, a sheep antibody, a chimeric antibody, a humanized antibody, and a human antibody. An antibody can be obtained as a polyclonal antibody or monoclonal antibody by known methods. In terms of stable production of uniform antibodies, a monoclonal antibody, and particularly, a monoclonal antibody derived from mammals is preferable. Such a monoclonal antibody derived from mammals may include those produced from hybridomas, and those produced from a host that is transformed with an expression vector containing an antibody gene by genetic engineering.

### (2) Hybridoma producing antibody

A hybridoma which produces the antibody used in the present invention can be basically prepared, for example, as follows, using known techniques. Briefly, using an IL-20Rβ protein or a cell expressing IL-20Rβ as a sensitizing antigen, a mammal is immunized by an ordinary immunization method. The obtained immunocytes are fused with known parent cells by an ordinary cell fusion method, and the fused cells is subjected to an ordinary screening method to screen monoclonal antibody-producing cells (hybridomas). Preparation of hybridomas can be carried out according to the method of Milstein *et al.* (Kohler. G. and Milstein, C., Methods Enzymol. (1981) 73: 3-46), for example. When the immunogenicity of an antigen is low, it may be bound to a macromolecule having immunogenicity, such as albumin, and immunization may be then carried out.

More specifically, first, IL-20Rβ used as a sensitizing antigen for obtaining an antibody can be obtained by expressing the gene/amino acid sequence encoding IL-20Rβ, which has already been disclosed in WO 99/46379, WO 00/53758, WO 00/39161, and WO 99/62934, and the like. A polynucleotide having a gene sequence encoding IL-20Rβ is inserted into a known expression vector system, and suitable host cells are transformed with the vector. Thereafter, an IL-20Rβ protein of interest is purified from the host cells or a culture supernatant thereof by known methods. Then, the purified IL-20Rβ protein or a partial peptide of the protein can be used as a sensitizing antigen. In addition, a partial peptide can also be obtained from the amino acid sequence of IL-20Rβ by chemical synthesis.

A mammal immunized with a sensitizing antigen is not particularly limited. It is preferable to select a mammal, taking into consideration compatibility with patent cells used in cell fusion. Generally, rodents, such as mice, rats, hamsters, rabbits, or monkeys can be used.

Immunization of an animal with a sensitizing antigen can be carried out by known methods. For example, a common method is intraperitoneal or subcutaneous injection of a sensitizing antigen to a mammal. More specifically, a sensitizing antigen is diluted with or suspended in PBS (phosphate-buffered saline), saline, or the like, into a suitable amount. Thereafter, a suitable amount of a common adjuvant, such as a Freund's complete adjuvant, is mixed with the obtained solution, as desired, followed by emulsification. The obtained product was administered to a mammal several times every 4 to 21 days. Moreover, a suitable carrier can also be used in immunization of a sensitizing antigen.

Thus, a mammal is immunized, and it is confirmed that a desired antibody level increases in the serum. Thereafter, immunocytes are collected from the immunized mammal and subjected to cell fusion. Spleen cells are particularly preferable as immunocytes.

Myeloma cells from mammals are used as parent cells to be fused with the above-described immunocytes. As myeloma cells, various known cell lines are used. Preferred examples of such myeloma cells may include P3(P3x63Ag8.653) (J. Immnol. (1979) 123, 1548-1550), P3x63Ag8U.1 (Current Topics in Microbiology and Immunology (1978) 81, 1-7), NS-1 (Kohler. G. and Milstein, C. Eur. J. Imunol, (1976) 6, 511-519), MPC-11 (Margulies. D. H. *et al.,* Cell (1976) 8, 405-415), SP2/0 (Shulman, M. *et al.,* Nature (1978) 276, 269-270), F0 (de St. Groth, S. F. *et al.,* J. Immunol. Methods (1980) 35, 1-21), S194 (Trowbridge, I. S. J. Exp. Med. (1978) 148, 313-323), and R210 (Galfre, G. *et al.,* Nature (1979) 277, 131-133).

Cell fusion between the above-described immunocytes and myeloma cells can be basically carried out according to known methods such as the method of Kohler and Milstein (Kohler G. and Milstein, C., Methods Enzymol: (1981) 73, 3-46).

More specifically, the above cell fusion is carried out in the presence of a cell fusion promoter in a common nutrient culture solution. For example, polyethylene glycol (PEG) or Sendai virus (HVJ) is used as a fusion promoter. In order to enhance fusion efficiency, as desired, auxiliary agents such as dimethyl sulfoxide can also be used.

The ratio between immunocytes and myeloma cells used can be suitably set. For example, immunocytes are preferably added to myeloma cells at a ratio between 1 : 1 and 10 : 1. Examples of a culture solution useful in the above cell fusion may include an RPMI1640 culture solution, an MEM culture solution, which are preferable for growth of the myeloma cells, and further, a common culture solution used in this type of cell culture. In addition, a serum infusion solution such as fetal calf serum (FCS) can also be used in combination.

For cell fusion, a certain amount of the above immunocytes and a certain amount of the above myeloma cells are fully mixed in the above culture solution. A PEG solution (for example, having a mean molecular weight between approximately 1,000 and 6,000), which has previously been heated to approximately 37°C, is added to the mixture generally in a concentration of 30% to 60% (w/v) and mixed, so as to form fused cells of interest (hybridomas). Subsequently, an operation to successively add a suitable culture solution followed by centrifugation to eliminate a supernatant is repeated, so as to eliminate a cell fusion promoter and the like, which are not preferable for the growth of hybridomas.

The thus obtained hybridomas are cultured in an ordinary selective culture solution such as an HAT culture solution (a culture solution containing hypoxanthine, aminopterin, and thymidine) for selection. The above culture in an HAT culture solution is continued for a time (generally several days to several weeks) enough for death of cells other than hybridomas of interest (non-fused cells). Subsequently, a common limiting dilution method is applied, and thus, hybridomas producing an antibody of interest are screened and subjected to monocloning.

In addition to the method of obtaining the above hybridomas by immunizing mammals other than a human with an antigen, several methods of obtaining a human antibody have also been known. For example, human lymphocytes are sensitized with IL-20Rβ or cells expressing IL-20Rβ *in vitro,* and the sensitized lymphocytes are then fused with myeloma cells derived from a human having a permanent division ability, so as to obtain a human antibody having activity to bind to IL-20Rβ (JP Patent Publication (Kokoku) No. 1-59878 B (1989)). Moreover, IL-20Rβ as an antigen is administered to a transgenic animal having the entire repertoire of human antibody genes, so as to obtain cells producing an anti-IL-20Rβ antibody. The cells are then subjected to immortalization, and a human antibody to IL-20Rβ may be obtained from the immortalized cells (see, WO 94/25585, WO 93/12227, WO 92/03918, and WO 94/02602).

Hybridomas producing the thus obtained monoclonal antibody can be subcultured in a common culture solution, and further, they can be stored in liquid nitrogen for a long time.

In order to obtain a monoclonal antibody from the hybridomas, a method of culturing the hybridomas according to an ordinary method to obtain an antibody as a culture supernatant, or a method of administering the hybridomas to a mammal compatible therewith and allowing them to grow therein so as to obtain an antibody as ascites, can be adopted. The former method is suitable for the case of obtaining an antibody with high purity. On the other hand, the latter method is suitable for mass production of antibodies.

### (3) Recombinant antibody

An antibody gene is cloned from hybridomas, and it can be then incorporated into a suitable vector. The vector is then introduced into a host, so that a recombinant antibody can be produced by genetic recombination techniques. Such a recombinant antibody can also be used (see, e.g., Carl, A. K. Borrebaeck, James, W. Larrick, THERAPEUTIC MONOCLONAL ANTIBODIES, Published in the United Kingdom by MACMILLAN PUBLISHERS LTD, 1990).

More specifically, cDNA in the variable region (V region) of an antibody is synthesized from mRNA of a hybridoma, using reverse transcriptase. For example, mRNA encoding the V region of an anti-IL-20Rβ antibody is isolated from a hybridoma producing the anti-IL-20Rβ antibody. mRNA can be isolated by known methods such as the guanidine ultracentrifugation method (Chirgwin, J. M. *et al.,* Biochemistry (1979) 18, 5294-5299) or the AGPC method (Chomczynski, P. *et al.,* Anal. Blochem. (1987) 162, 156-159). By such a method, total RNA is prepared, and mRNA of interest is then prepared using an mRNA Purification Kit (manufactured by Pharmacia) or the like. In addition, mRNA can also be directly prepared using a QuickPrep mRNA Purification Kit (manufactured by Pharmacia).

cDNA in the V region of an IL-20Rβ antibody is synthesized from the obtained mRNA, using reverse transcriptase. cDNA is synthesized using an AMV Reverse Transcriptase First-strand cDNA Synthesis Kit (manufactured by Seikagaku Corporation) or the like. Moreover, for synthesis and amplification of cDNA, the 5'-RACE method (Frohman, M. A. *et al.,* Proc. Natl. Acad. Sci. USA (1988) 85, 8998-9002. Belyavsky, A. *et al.,* Nucleic Acids Res. (1989) 17, 2919-2932) utilizing PCR can be applied, using a 5'-AmpliFINDER RACE Kit (manufactured by Clontech).

Once DNA encoding the V region of an IL-20Rβ antibody of interest is obtained, the DNA is ligated to DNA encoding a desired antibody constant region (C region), and the ligated DNA is incorporated into an expression vector. Otherwise, DNA encoding the V region of an antibody may be incorporated into an expression vector containing DNA in the C region of an antibody. DNA is incorporated into an expression vector such that it is expressed under control of expression control regions such as an enhancer and a promoter. Thereafter, host cells are transformed with the expression vector, so that an antibody is expressed therein.

For expression of an antibody gene, DNA fragments encoding either the heavy chain (H chain) or light chain (L chain) of an antibody may be separately incorporated into different expression vectors, so that host cells may be simultaneously transformed with these vectors. Otherwise, DNA fragments encoding either the H chain or the L chain are incorporated into a single expression vector, so that host cells may be transformed with the vector (see, WO 94/11523).

Moreover, a technique of obtaining a human antibody by panning with an antibody library of mammals such as a mouse or human has been known. For example, the variable region of a human antibody is expressed as a single chain antibody (scFv) on the surface of a phage by the phage display method, so as to select a phage binding to an antigen. The selected phage is subjected to gene analysis, so that a DNA sequence encoding the variable region of a human antibody binding to an antigen can be determined. If the DNA sequence of scFv binding to an antigen is clarified, a suitable expression vector containing the sequence is prepared, and a human antibody can be obtained. These methods have already been well known, and WO 92/01047, WO 92/20791, WO 93/06213, WO 93/11236, WO 93/19172, WO 95/01438, and WO 95/15388 can be used for references.

### (4) Modified antibody

In the present invention, an artificially modified gene recombinant antibody such as a chimeric antibody or humanized antibody can be used for the purpose of decreasing heterologous antigenicity to humans. These modified antibodies can be produced by known methods.

A chimeric antibody is an antibody, which comprises the variable region of the heavy chain and light chain of the antibody of a mammal other than a human, such as a mouse antibody, and the constant region of the heavy chain and light chain of a human antibody. DNA encoding the variable region of a mouse antibody is ligated to DNA encoding the constant region of a human antibody, and the ligated DNA is then incorporated into an expression vector. The expression vector is then introduced into a host, so as to obtain a chimeric antibody from the host.

A humanized antibody is referred to also as a reshaped human antibody. It is an antibody obtained by transplanting the complementarity-determining region (CDR) of a mammal antibody other than a human antibody, such as a mouse antibody, into the complementarity-determining region of a human antibody. A general gene recombination technique therefor has been known (see, EP Patent Application Publication No. EP125023 and WO 96/02576).

More specifically, a DNA sequence designed such that CDR of a mouse antibody is ligated to the framework region (FR) of a human antibody is synthesized by the PCR method from several oligonucleotides prepared such that they have an overlapped portion at the terminus thereof. The obtained DNA is ligated to DNA encoding the constant region of a human antibody, and the ligated DNA is then incorporated into an expression vector. The expression vector is then introduced into a host, so as to obtain a humanized antibody (see, EP Patent Application Publication No. 239400, and WO 96/02576).

As FR of a human antibody ligated via a complementarity-determining region, one wherein the CDR forms a good antigen-binding portion is selected. If necessary, amino acids in the framework region of the variable region of an antibody may be substituted so that the complementarity-determining region of a reshaped human antibody forms an appropriate antigen-binding site (Sato, K. *et al.,* Cancer Res. (1993) 53, 851-856).

As the C regions of a chimeric antibody and a humanized antibody, those of a human antibody are used. For example, in the case of the H chain, Cγ1, Cγ2, Cγ3, and Cγ4 can be used, and in the case of the L chain, Cκ and Cλ can be used. Moreover, in order to improve the stability of an antibody or a production thereof, the C region of a human antibody may be modified.

A chimeric antibody comprises the variable region of an antibody derived from a mammal other than a human and the constant region derived from a human antibody. On the other hand, a humanized antibody comprises the complementarity-determining region of an antibody derived from a mammal other than a human and the framework region and C region derived from a human antibody. Since a humanized antibody has a low antigenicity in a human body, it is useful as an active ingredient of the therapeutic agent of the present invention.

### (5) Antibody variants

The antibody used in the present invention is not limited to the whole molecules of an antibody, but it may be a fragment of an antibody or a variant thereof as long as it binds to IL-20Rβ and inhibits the activity of IL-20Rβ. Either a bivalent antibody or monovalent antibody may also be included in the antibody of the present invention. For example, examples of an antibody fragment may include Fab, F(ab')2, Fv, Fab/c having a Fab and a complete Fc, and a single chain Fv (scFv) obtained by linking Fv of H chain to Fv of L chain using a suitable linker.

More specifically, an antibody is treated with an enzyme such as papain or pepsin to generate antibody fragments. Alternatively, a gene encoding such an antibody fragment is constructed, and it is then introduced into an expression vector, followed by expression in suitable host cells (see, e.g., Co, M. S. *et al.,* J. Immunol. (1994) 152, 2968-2976, Better, M. & Horwitz, A. H. Methods in Enzymology (1989) 178, 476-496, Academic Press, Inc., Plueckthun, A. & Skerra, A. Methods in Enzymology (1989) 178, 476-496, Academic Press, Inc., Lamoyi, E., Methods in Enzymology (1989) 121, 652-663, Rousseaux, J. *et al.,* Methods in Enzymology (1989) 121, 663-669, Bird, R. E. *et al.,* TIBTECH (1991) 9, 132-137).

scFv is obtained by linking the H chain V region of an antibody to the L chain V region thereof. In such scFv, the H chain V region is ligated to the L chain V region via a linker, and preferably a peptide linker (Huston, J. S. *et al.,* Proc. Natl. Acad. Sci. U.S.A. (1988) 85, 5879-5883). In scFv, H chain V region and L chain V region may be derived from any antibodies described in the present specification. As a peptide linker ligating V region, a certain single chain peptide consisting of 1 to 30 amino acid residues is used, for example.

Among DNA encoding the H chain or H chain V region of the above-described antibody and DNA encoding the L chain or L chain V region thereof, using, as a template, a DNA portion encoding the whole or a desired part of amino acid sequences thereof, a DNA fragment is amplified by the PCR method with a primer set corresponding to both termini thereof. Subsequently, DNA encoding a peptide linker portion is amplified, using a primer set which are designed such that both termini thereof are ligated to H chain and L chain, respectively, thereby obtaining DNA encoding scFv.

Once DNA encoding scFv is produced, an expression vector containing the DNA, and a host transformed with the expression vector can also be obtained according to conventional methods. Using the host, scFv can be obtained according to conventional methods.

These antibody fragments are obtained by first obtaining their genes, expressing them in hosts, and allowing the hosts to produce them, in the same manner as described above. The term "antibody" may also include these antibody fragments in the present invention.

An anti-IL-20Rβ antibody binding to various molecules such as polyethylene glycol (PEG) can also be used as an antibody variant. The term "antibody" may also include these antibody variants in the present invention. These antibody variants can be obtained by chemically modifying the obtained antibody. It is to be noted that a method of modifying an antibody has already been established in this field.

Moreover, the antibody used in the present invention may also be a bispecific antibody. Such a bispecific antibody may have antigen-binding sites recognizing different epitopes on an IL-20Rβ molecule. Otherwise, one antigen-binding site may recognize IL-20Rβ and the other antigen-binding site may recognize other cell surface molecules such as an IL-20 receptor α chain (hereinafter referred to as IL-20Rα) or interleukin-22 receptor (hereinafter referred to as IL-22R). Furthermore, the other antigen-binding site may be a surface molecule of immunocytes such as T cells or natural killer cells. In this case, immunocytes can directly act on cells expressing IL20-Rβ to cause injury specifically to carcinoma cells, so as to suppress the growth of the carcinoma cells. Such a bispecific antibody can be produced by binding HL pairs of two types of antibodies. Otherwise, it can also be obtained by fusing hybridomas producing different monoclonal antibodies so as to produce bispecific antibody-producing fused cells. Still further, a bispecific antibody can also be produced by genetic engineering.

### (6) Expression and production of recombinant antibody or modified antibody

When the antibody gene obtained as described above is introduced into a suitable host to produce an antibody, a suitable host and a suitable expression vector can be used in combination. When eukaryotic cells are used as host cells, animal cells, plant cells, and fungi can be used. As animal cells, (1) mammalian cells such as CHO, COS, myeloma, BHK (baby hamster kidney), HeLa, or Vero, (2) amphibian cells such as *Xenopus laevis* oocytes, (3) insect cells such as sf9, sf21, or Tn5, and other cells, have been known. As plant cells, cells derived from Nicotiana such as *Nicotiana tabacum* have been known. These cells may be subjected to callus culture. As fungi, yeasts including Saccharomyces such as *Saccharomyces serevisiae,* and filamentous fungi including Aspergillus such as *Aspergillus niger* have been known. When prokaryotic cells are used, there is a production system using bacterial cells. As bacterial cells, *Escherichia coli* (*E. coli*) and *Bacillus subtilis* have been known. An antibody gene of interest is introduced into these cells by transformation, and the transformed cells are cultured *in vitro* to obtain an antibody.

For example, in the case of mammalian cells, a commonly used useful promoter is bound to an antibody gene to be expressed, and then, poly A signal is operatively linked downstream of the 3'-side thereof, so that an antibody can be expressed. Examples of a promoter/enhancer may include a human cytomegalovirus immediate early promoter/enhancer.

Other examples of a promoter/enhancer useful in the expression of an antibody used in the present invention may include a virus promoter/enhancer such as retrovirus, polyoma virus, adenovirus, or simian virus 40 (SV40), and a promoter/enhancer derived from mammalian cells, such as human elongation factor 1α (HEF1α).

When an SV40 promoter/enhancer is used, gene expression can easily be carried out by the method of Mulligan *et al.* (Nature (1979) 277, 108). When an HEF1α promoter/enhancer is used, gene expression can easily be carried out by the method of Mizushima *et al.* (Nucleic Acids Res. (1990) 18, 5322).

In the case of *Escherichia coli,* a commonly used useful promoter, a signal sequence for secretion of an antibody, and an antibody gene to be expressed are operatively linked to one another, so that the gene can be expressed. Examples of a promoter may include a lacZ promoter and an araB promoter. When a lacZ promoter is used, gene expression can be carried out by the method of Ward *et al.* (Nature (1098) 341, 544-546; FASEB J. (1992) 6, 2422-2427). When an araB promoter is used, gene expression can be carried out by the method of Better *et al.* (Science (1988) 240, 1041-1043).

When an antibody is to be produced in a periplasm of *Escherichia coli,* a pelB signal sequence (Lei, S. P. *et al.,* J. Bacteriol. (1987) 169,4379) may be used as a signal sequence for secretion of an antibody. An antibody produced in the periplasm is separated, and thereafter, the structure of the antibody is refolded, as appropriate, for use.

As a replication origin, those derived from SV40, polyoma virus, adenovirus, bovine papilloma virus (BPV), etc. can be used. In addition, in order to amplify the number of gene copies in a host cell system, an expression vector may comprise, as a selective marker, an aminoglycoside transferase (APH) gene, a thymidine kinase (TK) gene, an *Escherichia coli* xanthine guanine phosphoribosyl transferase (Ecogpt) gene, a dihydrofolate reductase (dhfr) gene, etc.

The transformed host cells are cultured *in vitro* or *in vivo*, so as to allow them to produce an antibody of interest. The host cells are cultured according to known methods. For example, DMEM, MEM, RPMI1640, or IMDM can be used as a culture solution. A serum infusion solution such as fetal calf serum (FCS) can also be used in combination.

Moreover, in order to produce a recombinant antibody, not only the above-described host cells, but transgenic animals can also be used. For example, an anti-IL-20Rβ antibody gene is inserted into a gene encoding a protein specifically produced in milk (e.g., goat β-casein), so as to prepare a fused gene. A DNA fragment containing the fused gene into which the above antibody gene has been inserted is injected into a goat embryo, and the embryo is then transferred into a female goat. An anti-IL-20Rβ antibody is obtained from milk generated from a transgenic goat that was born from the goat accepting the above embryo, or progenies thereof. In addition, in order to increase the amount of the milk containing the anti-IL-20Rβ antibody generated from the transgenic goat, hormone may be added to the transgenic goat, as appropriate (see, Ebert, K. M. *et al.,* Bio/Technology (1994) 12, 699-702).

### (7) Separation and purification of antibody

The antibody expressed and produced as described above can be separated from cells or a host animal, and it can be purified until it becomes homogenous. The antibody used in the present invention can be separated and purified with an affinity column. Examples of a column using a protein A column may include Hyper D, POROS, and Sepharose F.F. (manufactured by Pharmacia). Further, commonly used protein separation and purification methods may be used, and such methods are not limited. For example, an antibody can be separated and purified by appropriately selecting and combining methods such as chromatography columns other than the above affinity column, filter, ultrafiltration, salting out, and dialysis (Antibodies A Laboratory Manual. Ed Harlow, David Lane, Cold Spring Harbor Laboratory, 1988). Examples of chromatography may include ion exchange chromatography, hydrophobic chromatography, and gel filtration.

### (8) Confirmation of activity of antibody

In order to determine the antigen-binding activity of the antibody used in the present invention, known methods can be applied (Antibodies A Laboratory Manual. Ed Harlow, David Lane, Cold Spring Harbor Laboratory, 1988). For example, ELISA (enzyme-linked immunosorbent assay), EIA (enzyme-linked immunoassay), RIA (radioimmunoassay), or the fluorescent antibody method can be used.

When ELISA is used, a sample containing an anti-IL-20Rβ antibody, such as the culture supernatant of anti-IL-20Rβ antibody-generating cells or a purified antibody, is added to a plate on which IL-20Rβ is coated. A secondary antibody labeled with enzyme such as alkaline phosphatase is added to the plate. The plate is then incubated and washed. Thereafter, an enzyme substrate such as p-nitrophenyl phosphate is added thereto, so that antigen-binding activity can be evaluated by measuring absorbance.

### (9) Cytotoxicity

The anti-IL-20Rβ antibody used in the present invention has cytotoxicity such as antibody-dependent cellular cytotoxicity (ADCC) or complement-dependent cytotoxicity (CDC). The ADCC activity or CDC activity of the antibody obtained by the above method can be determined by a measurement method described below, for example.

ADCC activity can be determined by mixing effector cells, target cells, and an anti-IL-20Rβ antibody and examining the level of ADCC. As effector cells, for example, mouse spleen cells, or monocytes isolated from human peripheral blood or bone marrow can be used. As target cells, human lung carcinoma cell lines such as A549, NCI-H460, NCI-H23, or NCI-522 can be used. Target cells have previously been labeled with ⁵¹Cr, and an anti-IL-20Rβ antibody is added to the cells, followed by incubation. Thereafter, effector cells are added to the target cells at an appropriate ratio, followed by incubation. After the incubation, the culture supernatant is collected, and radioactivity in the supernatant is counted, so as to determine ACDD activity.

CDC activity can be determined by mixing the above labeled target cells with an anti-IL-20Rβ antibody, adding a complement to the mixture followed by incubation, and then counting radioactivity in the supernatant after the culture.

### (10) Neutralizing activity on IL-20Rβ

The anti-IL-20Rβ antibody used in the present invention has activity of blocking signals that can be transduced into cells via IL-20Rβ (neutralizing activity on IL-20Rβ). The neutralizing activity of the antibody obtained by the above method on IL-20Rβ can be determined by a measurement method described below, for example.

An IL-20R gene is introduced, singly or together with an appropriate gene such as an IL20Rα gene or IL-22R gene, into cells growing depending on IL-3, such as mouse Ba/F3, so as to establish a cell line capable of growing depending on signals via IL-20Rβ due to IL-20, IL-19, IL-24, or the like, instead of signals via IL-3.

During the establishment of the cell line, a chimeric receptor obtained by substituting the intracellular region of each receptor into, for example, the intracellular region of a G-CSF receptor is used, so that signals can be more effectively transduced.

Such a cell line is cultured in the presence of IL-20, IL-19, IL-24, or the like, and the antibody used in the present invention is then added thereto to examine the ability of the antibody to suppress the growth of the cells, thereby determining the "activity of blocking signals via IL-20Rβ" of the antibody.

Otherwise, using a human carcinoma cell line capable of growing depending on signals via IL-20Rβ due to IL-20, IL-19, IL-24, or the like, and preferably using a human lung carcinoma cell line, the "activity of blocking signals via IL-20Rβ" of the antibody can be determined by the same method described above.

Moreover, the "activity of blocking signals via IL-20Rβ" of the antibody can also be determined by detecting intracellular signals transduced via IL-20Rβ, such as activation of STAT3. To detect intracellular signals, a detection system using a reporter gene can be used, for example. Such a reporter gene can be produced, for example, by ligating a sequence whose transcription activity is promoted by activated STAT3 (STAT3-reactive sequence) to an appropriate reporter. As a STAT3-reactive sequence, a consensus sequence to which STAT3 reportedly binds, or a promoter of c-fos gene whose expression is reportedly controlled by STAT, can be used. A commonly used firefly luciferase gene can be used as a reporter. The activity of the expressed luciferase can be determined by a common method, for example, using Dual-Luciferase^{R} Reporter Assay System (Promega Corporation). A reporter gene is introduced into appropriate cells such as A549 cells, HEK293 cells, or BHK cells. Thereafter, the cells are stimulated with a ligand such as IL-20, IL-19, IL-24, or the like, to measure of the expression of the reporter. The antibody is simultaneously added to such a system, so that the "activity of blocking signals via IL-20Rβ" of the antibody can be determined. In addition, together with a reporter gene, a gene constituting a receptor, such as an IL-20Rβ gene, IL-20Rα gene, or IL-22R gene, may also be simultaneously introduced.

Furthermore, the inhibitory activity of the antibody on a ligand such as IL-20, IL-19, or IL-24 physically binding to a receptor complex containing IL-20Rβ is determined, so that the "activity of blocking signals via IL-20Rβ" of the antibody can also be determined.

In order to determine "the physical binding of a ligand such as IL-20, IL-19, or IL-24 to a receptor complex containing IL-20Rβ," a sensor chip onto which a receptor protein or ligand protein is immobilized is used, and surface plasmon resonance (SPR) is detected with a device such as BIACore (Amersham Biosciences).

Alternatively, using a ligand labeled with a radioisotope, enzyme, or biotin, the binding of the labeled ligand to an immobilized receptor protein is detected by the above-described ELISA method, EIA method, RIA method, or the like, so as to determine the physical binding of a ligand to a receptor complex containing IL-20Rβ.

### 3. Pharmaceutical composition containing antibody

A pharmaceutical composition which comprises, as an active ingredient, an anti-IL-20Rβ antibody capable of binding to the β chain of an IL-20 receptor, obtained by the above-described method can be used for the purpose of treating lung carcinoma.

In addition, a pharmaceutical composition comprising, as an active ingredient, an anti-IL-20Rβ antibody to which a radioisotope, chemotherapeutic, or cellular cytotoxic substance such as bacteria-derived toxin is bound, can also be used for the purpose of treating lung carcinoma.

It has been known that IL-20Rβ forms a heterodimer together with IL-20Rα or IL-22R to transduce signals into cells (J Immunol. 2001 Oct 1; 167(7): 3545-9). Thus, even if any one of these is targeted, signals via IL-20Rβ can be blocked. Accordingly, a pharmaceutical composition may comprise, as an active ingredient, not only the anti-IL-20Rβ antibody, but it may also comprise antibodies capable of specifically binding to IL-20Rα or IL-22R, alone or in combination. However, it is considered that the case of targeting IL-20Rβ showing a limited expression pattern in normal tissues has a less influence on normal cells, and that it is more advantageous in view of reduction in side effects. Accordingly, a pharmaceutical composition may preferably comprise an anti-IL-20Rβ antibody as an active ingredient.

The pharmaceutical composition of the present invention can be administered by parenteral systemic route or by local route. Examples of a specific administration method may include intravenous injection, intramuscular injection, intraperitoneal injection, subcutaneous injection, and transpulmonary administration. Such an administration method can be selected, as appropriate, depending on the age, symptoms, or the like of a patient. An effective dosage is selected within the range between 0.001 mg and 1,000 mg per kg of body weight per dose. Otherwise, the dosage may be between 0.01 mg and 100,000 mg per patient. However, the dosage is not limited thereto.

In addition, with regard to the timing for administration of the pharmaceutical composition of the present invention, it can be administered either before or after the development of the clinical symptoms of the disease.

The pharmaceutical composition of the present invention can be formulated according to conventional methods (Remington's Pharmaceutical Science, latest edition, Mark Publishing Company, Easton, U.S.A.). The formulation may comprise pharmaceutically acceptable carriers or additives.

Examples of such a carrier or additive may include water, a pharmaceutically acceptable organic solvent, collagen, polyvinyl alcohol, polyvinylpyrrolidone, a carboxyvinyl polymer, carboxymethylcellulose sodium, sodium polyacrylate, sodium alginate, soluble dextran, carboxymethylstarch sodium, pectin, methylcellulose, ethylcellulose, xanthan gum, gum Arabic, casein, gelatin, agar, diglycerine, propylene glycol, polyethylene glycol, Vaseline, paraffin, stearyl alcohol, stearic acid, human serum albumin (HSA), mannitol, sorbitol, lactose, and a surfactant acceptable as a pharmaceutical additive.

Practically used additives are appropriately selected from the above-listed additives, singly or in combination, depending on a dosage form. However, additives are not limited thereto. When the pharmaceutical composition of the present invention is used as a formulation for injection, for example, a purified anti-IL-20Rβ antibody is dissolved in a solvent such as a saline, buffer, or glucose solution. An anti-adsorption agent such as Tween 80, Tween 20, gelatin, or human serum albumin is then added to the solution for use. Alternatively, in order to obtain a dosage form that is dissolved and reconstructed before use, the formulation may also be freeze-dried. Examples of an excipient used in freeze-drying may include sugar alcohols such as mannitol or glucose and sugars.

### 4. Pharmaceutical composition comprising antisense polynucleotide as active ingredient

In the present invention, a pharmaceutical composition comprising, as an active ingredient, an antisense polynucleotide to a polynucleotide encoding IL-20Rβ, which can inhibit the expression of IL-20Rβ, can be used for the purpose of treating lung carcinoma.

The term "antisense polynucleotide" is used herein to mean not only those wherein nucleotides corresponding to nucleotides constituting a certain region of DNA or mRNA are all complementary thereto. But the nucleotides may contain some mismatches, as long as the DNA or mRNA can stably hybridize with the oligonucleotide. The synthesis method or origin of the antisense polynucleotide is not limited.

The antisense polynucleotide used for the pharmaceutical composition of the present invention is preferably an antisense polynucleotide to a nucleotide sequence consisting of 15 to 30 contiguous nucleotides containing a 5' region encoding IL-20Rβ.

These antisense polynucleotides can be obtained by conventional methods. For example, they are easily synthesized with a commercially available DNA synthesizer. Synthesis of the antisense polynucleotides can be achieved by a solid phase synthesis method using phosphoroamidite, or solid phase synthesis method using hydrogen phosphonate. Purification and confirmation of the purity can be carried out by the high performance liquid chromatography and the polyacrylamide gel electrophoresis. The molecular weight can be confirmed by the Electrospraylonization Mass Spectrometry or the Fast Atom Bonbardment-Mass Spectrometry. The synthesis method or origin of the antisense polynucleotide used for the pharmaceutical composition of the present invention is not limited, as long as it has a sequence hybridizing with the nucleotide sequence of DNA or mRNA encoding IL-20Rβ.

The antisense polynucleotide obtained as described above acts on IL-20Rβ-producing cells, and binds to DNA or mRNA encoding IL-20Rβ, so that it inhibits the transcription or translation thereof and suppresses the expression of IL-20Rβ. Thus, the antisense polynucleotide has an effect to suppress the action of IL-20Rβ. Accordingly, a pharmaceutical composition comprising, as an active ingredient, an antisense polynucleotide to IL-20Rβ is useful for treatment of lung carcinoma.

With regard to the pharmaceutical composition of the present invention comprising an antisense polynucleotide as an active ingredient, an appropriate base is mixed with the antisense polynucleotide to obtain an external preparation such as a liniment. In addition, an excipient, isotonizing agent, solubilizing agent, stabilizing agent, antiseptic, soothing agent, or the like is added to the pharmaceutical composition, as necessary, so as to form a tablet, powder, granule, capsule, liposome capsule, injectable solution, liquid formulation, nasal drop, freeze-drying agent, etc. These agents can be prepared by conventional methods.

The pharmaceutical composition of the present invention containing an antisense polynucleotide is applied to patients by directly applying on the affected area of the patients, or administering into the blood vessel thereby reaching the affected area. Further, an antisense-encapsulating material can also be used to enhance sustainability and membrane permeability. Examples of such an antisense encapsulating material may include liposome, poly-L-lysine, lipid, cholesterol, lipofectyl, and their derivatives. The dosage of the pharmaceutical composition of the present invention containing an antisense polynucleotide is adjusted, as appropriate, depending on the state of a patient, and thus, the pharmaceutical composition can be used in a preferred amount. For example, it can be administered at dosages within the range between 0.1 and 100 mg/kg, and preferably between 0.1 and 50 mg/kg.

### 5. Diagnostic agent containing anti-IL-20Rβ antibody used for lung carcinoma, and detection method

The diagnostic agent containing an anti-IL-20Rβ antibody used in the present invention can be used to diagnose lung carcinoma of mammals (humans, etc.). More specifically, the present invention provides a diagnostic agent for diagnosing lung carcinoma, which detects an antigen (IL-20Rβ) in a test sample by immunoassay using an anti-IL-20Rβ antibody, and a detection method of lung carcinoma. The antibody may be either a monoclonal antibody or polyclonal antibody, as long as it is capable of binding to IL-20Rβ. In addition, a human antibody, humanized antibody, chimeric antibody, or the like can be used, as appropriate. Examples of immunoassay may include, but are not limited to, enzyme immunoassay, radioimmunoassay, fluoroimmunoassay, luminescence immunoassay, and immunoprecipitation method. Examples of a test sample available for the diagnosis of the present invention may include body fluid such as lymph, blood, and synovial fluid, and lung tissues collected by biopsy.

The method of the present invention includes detection of the expression of IL-20Rβ in a sample obtained from mammals being likely to have lung carcinoma, and particularly from a human. For example, a test sample obtained from a subject being potentially to have lung carcinoma is bound to an anti-IL-20Rβ antibody. Then, the amount (expression level) of IL-20Rβ complex in the antibody and the test sample is detected, and the obtained value is compared with a standard value, so as to confirm the existence of lung carcinoma. Herein, as a "standard value," a statistic value obtained by measuring the expression level in a large number of normal samples may be used, or the expression level in the normal tissues of a single subject may also be used as a standard value. An increase in the expression of IL-20Rβ genes does not occur due to differences among individuals, but it is a phenomenon occurring due to canceration of lung tissues of a single patient. Thus, a sample obtained from tissues other than lung can be used as a control sample. A person skilled in the art can appropriately determine a standard value (control sample) to be used in diagnosis, based on the descriptions in the present specification and knowledge in the present field. Thus, a standard value is not particularly limited.

Using nucleic acid amplification methods such as the PCR method, the expression of IL-20Rβ can also be detected in a test sample. More specifically, using, as a template, cDNA derived from mammals being likely to have lung carcinoma, and particularly derived from lung tissues of a human, expression analysis is carried out by PCR with primers specific for the IL-20Rβ gene. Thereby, it is possible to detect lung carcinoma even at the early stage where no symptoms are clinically found. As a control, normal tissues derived from a single subject or different subjects can be used. Alternatively, a standard normal level, which has previously been determined, can also be used as a control.

Moreover, an anti-IL-20Rβ antibody labeled with a radioisotope (¹²⁵I, ¹³¹I, ⁹⁰Y, etc.) is intravenously administered to a subject, so as to detect not only initial lung carcinoma but also localization of metastatic lung carcinoma.

### EXAMPLES

The present invention will be specifically described in the following examples. However, the examples are not intended to limit the scope of the present invention.

### [Example 1] Subtraction

Subtraction was carried out basically according to the method of Luda Diatchenko *et al..* (Proc Natl Acad Sci U.S.A., 1996, Jun 11; 93(12): 6025-30), using a PCR-Select™ cDNA Subtraction kit (CLONTECH).

According to a standard method using MMLV reverse transcriptase, a double-stranded cDNA was synthesized from each of human squamous cell lung carcinoma sample-derived polyA⁺ RNA (Invitrogen), human normal lung-derived polyA⁺ RNA (Invitrogen), normal human bone marrow-derived polyA⁺ RNA (CLONTECH), and normal human thymus gland-derived polyA⁺ RNA (CLONTECH). Thereafter, the termini of the obtained double-stranded cDNA were blunt-ended with T4 DNA polymerase and were then cleaved with RsaI.

A portion of cDNA (tester) derived from the human squamous cell lung carcinoma sample was divided into two portions. Thereafter, the two portions were ligated to adaptor 1 consisting of two types of polynucleotides having nucleotide sequences shown in SEQ ID Nos. 1 and 2, and adaptor 2 consisting of two types of polynucleotides having nucleotide sequences shown in SEQ ID Nos. 3 and 4, separately. A mixture (driver) consisting of normal human lung-derived cDNA, normal human bone marrow-derived cDNA, and normal human thymus gland-derived cDNA in excess was added to the sample containing a portion of the cDNA (tester) derived from the human squamous cell lung carcinoma sample ligated to each adaptor. Thereafter, heat denaturation was carried out thereon, and the first hybridization was then carried out at 68°C for 8 hours.

Subsequently, solutions obtained after the hybridization were mixed without performing heat denaturation, and an excessive amount of the heat-denatured driver was added to the mixture, followed by the second hybridization at 68°C for about 16 hours. Thereafter, the resultant product was diluted with a dilution buffer, and the diluted product was incubated at 75°C for 7 minutes. cDNA obtained by eliminating a shorter strand of the above adaptors shown in SEQ ID Nos. 2 and 4 was used as a template for PCR. PCR (suppression PCR) was carried out, using PCR primers 1 and 2, respectively, shown in SEQ ID Nos. 5 and 6, which were primers corresponding to each adaptor, so that cDNA (subtracted cDNA) having different adaptors at both termini was selectively amplified. A portion thereof was used as a template, and PCR was carried out using Nested PCR primers 1 and 2, respectively, shown in SEQ ID Nos. 7 and 8, which were primers located further inside the PCR primers 1 and 2, so as to obtain a product with higher selectivity.

The obtained product was purified with a QIAquick PCR Purification kit (QIAGEN), and the purified product was then cloned into a pT7Blue-T vector (Novagen) by the TA cloning method.

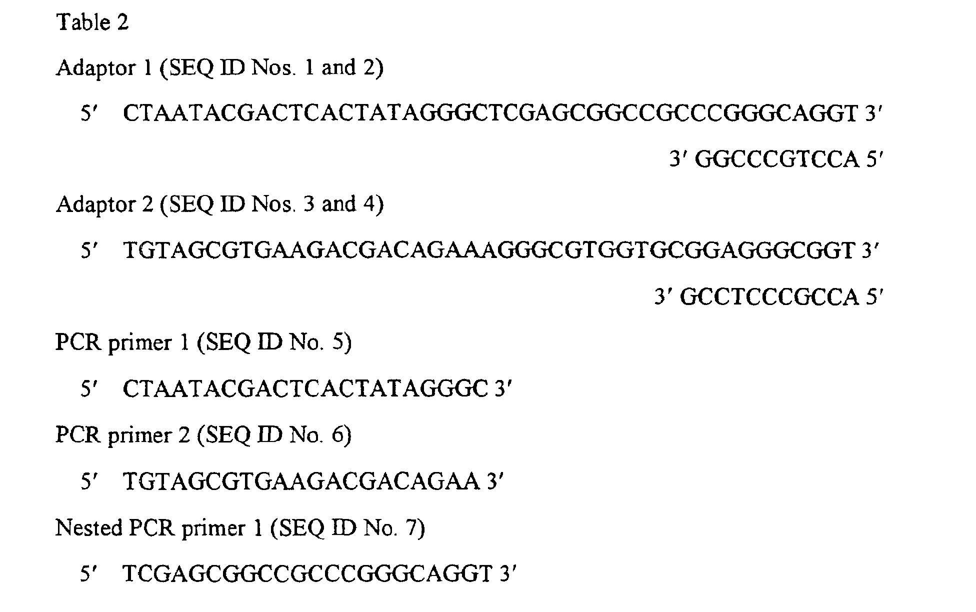

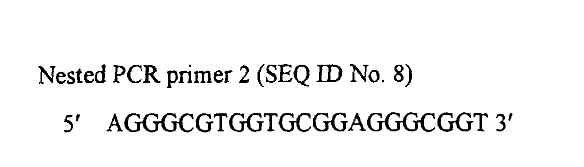

### [Example 2] Determination of sequence

Sequencing was carried out by the cycle sequencing method using, as a template, plasmid DNA prepared by alkaline-SDS method or a PCR product obtained by colony PCR, and using ABI PRISM™ Dye Terminator Cycle Sequencing Ready Reaction Kit with AmplyTaq DNA Polymerase, FS (Perkin Elmer). The sequence was determined using an ABI 377 DNA Sequencer.

Colony PCR was carried out by directly suspending colonies containing recombinant in a PCR reaction solution containing, as vector primers, M13 P4-22 primer (SEQ ID No. 9; 5' CCAGGGTTTTCCCAGTCACGAC 3') and M13 P5-22 primer (SEQ ID No. 10; 5' TCACACAGGAAACAGCTATGAC 3'). After completion of the PCR reaction, unreacted primers, nucleotides, and others were eliminated from the amplified insert DNA by the gel filtration method or the like, and the residual product was used as a template for sequencing.

### [Example 3] RT-PCR analysis

A single-stranded cDNA was synthesized from polyA⁺ RNA by a standard method using SUPERSCRIPT™ II RNase H- Reverse Transcriptase (GIBCO BRL). A portion of the single-stranded cDNA was used as a template for RT-PCR. PCR was basically carried out under the following conditions:

### 1. Composition of reaction solution

| | |
|---|---|
| TaKaRa ExTaq (TaKaRa) | 0.3 µL |
| TaqStart™ Antibody (CLONTECH) | 0.3 µL |
| 10 x ExTaq buffer (TaKaRa) | 3 µL |
| 2.5 mM dNTPs (TaKaRa) | 2.4 µL |
| 20 µM primers | 0.6 µL each |
| template DNA | 3 µL |
| Water was added such that the total amount became | 30 µL. |

### 2. Reaction conditions

94°C, 2 minutes → 28 to 35 cycles x (94°C, 30 seconds; 50°C to 60°C, 1 minute; and 72°C, 1 minute) → 72°C, 3 minutes
94°C, 2 minutes → 28 to 35 cycles x (94°C, 30 seconds; and 68°C, 2 minutes) → 72°C, 3 minutes

### 3. Primers

(1) Primers concerning CRA262
   CRA262-1 primer (SEQ ID No. 11) 5' CCACATTGGGCTCACAGACCTCAG 3'
   CRA262-2 primer (SEQ ID No. 12) 5' GTAGGCCACAAGGAACTCAAACTG 3'
(2) Primers concerning RP S9
   Upstream primer (SEQ ID No. 13) 5' GATGAGAAGGACCCACGGCGTCTGTTCG 3'
   Downstream primer (SEQ ID No. 14)

5' GAGACAATCCAGCAGCCCAGGAGGGACA 3'

The obtained results are shown in Figure 2. In the figure, the upper case shows the expression of a CRA262 (IL-20Rβ) gene, and the lower case shows the expression of a ribosome protein S9 gene (RP S9) as a control. As is clear from Figure 2, the expression of the CRA262 (IL-20Rβ) gene was extremely low in normal cells, but a significant increase in the expression of the gene was observed in carcinoma cells. Accordingly, it became clear that the CRA262 (IL-20Rβ) gene is deeply associated with the growth of lung carcinoma cells.

### [Example 4] Analysis of expression profile of various types of molecules (IL-20Rβ-associated molecules) associated with signal transduction via IL-20Rβ in non small cell lung carcinoma clinical samples

PolyA⁺ RNA was prepared from each of carcinoma tissues derived from patients with 4 different types of non small cell lung carcinoma and the tissues of normal lung, liver, thymus gland, bone marrow, and small intestine. A single-stranded cDNA was synthesized from the polyA⁺ RNA under the conditions described in Example 1. A portion of the single-stranded cDNA was used as a template, and expression analysis was carried out by PCR using primers specific for genes described below. PCR conditions described in Example 3 were basically applied, and the conditions were modified, as appropriate.
(1) Primers to IL-20Rα
   C80 primer (SEQ ID No. 17) 5' cag gag gaa gag gag gtg aaa cat 3'
   C81 primer (SEQ ID No. 18) 5' tct tgg agc tga ggg gtg tat gag 3'
(2) Primers to IL-20Rβ
   CRA262-1 primer (SEQ ID No. 11)
   CRA262-2 primer (SEQ ID No. 12)
(3) Primers to IL-22R
   C86 primer (SEQ ID No. 19) 5' tcc atg ggc ttc ctc gtc gca gta 3'
   C87 primer (SEQ ID No. 20) 5' ggg cag cgt ttg ggg cat agg aca 3'
(4) Primers to IL-19
   C62 primer (SEQ ID No. 21) 5' atc agg agc caa acc cca aaa tct 3'
   C63 primer (SEQ ID No. 22) 5' ctt ccc cat ctc ctt ccc ctt caa 3'
(5) Primers to IL-20
   C68 primer (SEQ ID No. 23) 5' gtg caa gcc aaa gat gga aac att 3'
   C69 primer (SEQ ID No. 24) 5' tat tct gtc tcc tcc atc cat tgc 3'
(6) Primers to IL-24
   C72 primer (SEQ ID No. 25) 5' aga ccc ttc tgc cct cct ttg ctg 3'
   C73 primer (SEQ ID No. 26) 5' agt tgt tgg cca gag tag aga atg 3'
(7) Primers to RP S9
   Upstream primer (SEQ ID No. 13)
   Downstream primer (SEQ ID No. 14)

As a result, it was revealed that among IL-20Rα, IL-20β, and IL-22R that were subunits for forming IL-19, IL-20, and IL24 receptors, the expression of IL-20Rβ was only significantly increased with canceration of the lung (Figure 3). From this result, it was found that among these receptor subunits, IL-20Rβ is particularly deeply associated with canceration of the lung.

In addition, it was also found that among the 3 types of ligands, in particular, the gene expression of IL-24 was highly increased in lung carcinoma cells (Figure 4). From this result, it is considered that signals transduced by IL-24 play a role extremely important for the growth of lung carcinoma cells (Figure 5).

On the other hand, it was clarified that the gene expression of IL-19 and IL-20 other than IL-24 was observed in lung carcinoma cells (Figure 4). In the case of these genes, the gene expression was not significantly increased with canceration. However, since the expression was observed in lung carcinoma cells, it is considered that IL-19 and IL-20 are deeply associated with the growth of lung carcinoma cells. In particular, since it had been clarified that IL-20 plays an important role in the growth of skin keratinocytes, it is assumed that signals from IL-20 play an important role also for the growth of lung carcinoma cells. It was found by the present invention that IL-20Rβ is a receptor, which is commonly associated with signal transduction of all of these ligands (Figure 5), and that the expression of IL-20Rβ is significantly increased during canceration of the lung. Accordingly, it is considered that an agent for targeting IL-20Rβ and blocking the signals thereof can efficiently suppress the growth of lung carcinoma cells.

Subsequently, using, as a template, cDNA derived from tumor cells (T) and non-tumor cells (N) of 5 patients with lung carcinoma (Matched cDNA Pair, CLONTECH), expression analysis was carried out by PCR with primers specific for genes described below. PCR conditions described in Example 3 were basically applied, and the conditions were modified, as appropriate.
(1) Primers to IL-20Rβ
   CRA262-1 primer (SEQ ID No. 11)
   CRA262-2 primer (SEQ ID No. 12)
(2) Primers to RP S9
   Upstream primer (SEQ ID No. 13)
   Downstream primer (SEQ ID No. 14)

As a result, it was clarified that the expression of the IL-20Rβ gene was significantly increased in tumor cells of 4 patients with lung carcinoma (Figure 6). From the result, it was confirmed that an increase in the expression of the IL-20Rβ gene in lung carcinoma cells, which had been confirmed by the previous RT-PCR analysis, does not occur due to differences among individuals, but that it is a phenomenon occurred due to canceration of lung tissues of a single patient.

### [Example 5] Analysis of gene expression profile of IL-20Rβ-associated molecules in human non small cell lung carcinoma cell lines

Total RNA was prepared from the following 10 human non small cell lung carcinoma cell lines using ISOGEN (Nippongene) in accordance with a standard method recommended by the manufacturer.

### <Human non small cell lung carcinoma cell lines>

### LC-1/sq, EBC-1, LK-2, RERF-LC-AI, LC-2/ad, ABC-1, A549, NCI-H460, NCI-H23, and NCI-H522

From the thus obtained total RNA, a single-stranded cDNA was synthesized using a SUPERSCRIPT™ First-Strand Synthesis System for RT-PCR (Invitrogen) in accordance with a standard method recommended by the manufacturer. Oligo dT (12 to 18 mer) was used as primers for a reverse transcription reaction. Using a portion of the cDNA as a template, PCR analysis was carried out with primers specific for genes described below. PCR conditions described in Example 3 were basically applied, and the conditions were modified, as appropriate.
(1) Primers to IL-20Rα
   C78 primer (SEQ ID No. 27) 5' tat ggg caa aag aaa tgg ctg aat 3'
   C79 primer (SEQ ID No. 28) 5' ggg acc acg ttc tgt ttg att tag 3'
(2) Primers to IL-20Rβ
   B93 primer (SEQ ID No. 29) 5' cca aca tga agc atc tct tga t 3'
   B96 primer (SEQ ID No. 30) 5' ccc cca ctc ctc acc att ttg a 3'
(3) Primers to IL-22R
   C84 primer (SEQ ID No. 31) 5' ttc atc cta ccc cca cgc caa tcc 3'
   C85 primer (SEQ ID No. 32) 5' agg tgc agg cgg ctt ggt gac ata 3'
(4) Primers to IL-19
   C64 primer (SEQ ID No. 33) 5' aag cca cca atg cca cca gag tca 3'
   C65 primer (SEQ ID No. 34) 5' cag gcc ctt tca gca gat aga gtt 3'
(5) Primers to IL-20
   C11 primer (SEQ ID No. 35) 5' agt gtg caa gcc aaa gat gga a 3'
   C14 primer (SEQ ID No. 36) 5' ctg gct gta ttt ctt cat tgc t 3'
(6) Primers to IL-24
   C76 primer (SEQ ID No. 37) 5' gat gct gag agc tgt tac ctt gtc 3'
   C77 primer (SEQ ID No. 38)

5' aaa tga cac agg gaa caa acc agt 3'

As a result, it was revealed that the expression of the IL-20Rβ gene was observed in all of the examined 10 non small cell lung carcinoma cell lines (Figure 7). In addition, it was also found that the gene expression of IL-20α and IL-22, which are known to form a heterodimer with IL-20Rβ, was observed at a high frequency. From these results, it was considered that a receptor consisting of IL-20Rβ/IL-20Rα or IL-20Rβ/IL-22R is expressed in lung carcinoma cells at a high frequency, and that the receptor is deeply associated with the growth of lung carcinoma cells. Subsequently, the gene expression of IL-19, IL-20, and IL-24, which are ligands of these receptors, was examined. As a result, it was clarified that these were also expressed in non small cell lung carcinoma cell lines at a high frequency (Figure 8). In particular, in LC-1/sq, EBC-1, RERF-LC-AI, LC-2/ad, A549, NCI-H460, and NCI-H23, any of ligands, and either IL-20Rβ/IL-20Rα or IL-20Rβ/IL-22R, are expressed together. Accordingly, it was considered that an autocrine mechanism due to these ligands and receptors is likely to function.

### [Example 6] Analysis of expression profile of IL-20Rβ gene in other types of carcinomas

A portion of cDNA derived from tumor cells (T) and non-tumor cells (N) from 4 patients with gastric carcinoma (S1, S3, S4, and S5) (Matched cDNA Pair, CLONTECH) and a portion of cDNA derived from tumor cells (T) and non-tumor cells (N) from 4 patients with uterine carcinoma (U1, U4, U5, and U6) (Matched cDNA Pair, CLONTECH) were used as templates. PCR analysis was carried out with primers specific for genes described below. PCR conditions described in Example 3 were basically applied, and the conditions were modified, as appropriate.
(1) Primers to IL-20Rβ
   B93 primer (SEQ ID No. 29)
   B96 primer (SEQ ID No. 30)
(2) Primers to RP S9
   Upstream primer (SEQ ID No. 13)
   Downstream primer (SEQ ID No. 14)

As a result, it was revealed that no significant increase in the expression of IL-20Rβ in tumor cells was observed in any patients (Figure 9). From this result, it was considered that the involvement of signals via IL-20Rβ in canceration or the growth of carcinoma cells is extremely low in the case of gastric carcinoma or uterine carcinoma. This is to say, it was found that signals via IL-20Rβ are not associated with canceration or the growth of carcinoma cells in all types of carcinomas, but that the signals play an important role only in several types of carcinomas such as lung carcinoma. Accordingly, by the present invention, it was clarified that signals via IL-20Rβ play an important role for canceration or the growth of carcinoma cells, especially in lung carcinoma, and thereby usefulness of the agent targeting IL-20Rβ was discovered.

### [Example 7] Preparation of expression plasmid of L-20Rβ-associated molecules

A plasmid expressing various types of IL-20Rβ-associated molecules was prepared as follows. First, using primers specific for genes described below, cDNA of interest was amplified from each template DNA by the PCR method. The conditions described below or PCR conditions described in Example 3 were basically applied, and the conditions were modified, as appropriate. The amplified cDNA was cleaved with restriction enzymes of which site had been added to primers, and the cleaved cDNA was incorporated into a suitable site of an expression vector (pCXND3) for a mammalian cell. By sequencing a plurality of clones, a clone in which cDNA was incorporated in a correct direction with no PCR errors was selected and used in the sequent experiments.
- Name of plasmid:: pCXND3/hIL-20Rβ
- Template:: human lung carcinoma-derived cDNA pool
- Encoded Protein:: human IL-20Rβ, entire ORF, no tags
- Primers:: D7 5' gat gaa ttc gaa gaa aga tgg ctg aga tgg aca 3' (SEQ ID No. 39) D8 5' gat gaa ttc tgg cgg aaa aca gaa aca caa ctt 3' (SEQ ID No. 40)
- Name of plasmid:: pCXND3/hsIL-20Rβ-FLAG
- Template:: pCXND3/hIL-20Rβ
- Encoded Protein:: human IL-20Rβ, only extracellular region, C-terminal FLAG tag added
- Primers:: D15 5' gat gaa ttc tcc acc atg cag act ttc aca atg 3' (SEQ ID No. 41) D16 5' gat gaa ttc tta ctt gtc atc gtc gtc ctt gta gtc ctc tcc ttg cac ctc cac 3' (SEQ ID No. 42)
- Name of plasmid:: pCXND3/hsIL-20Rβ-His
- Template:: pCXND3/hIL-20Rβ
- Encoded Protein:: human IL-20Rβ, only extracellular region, C-terminal His tag added
- Primers:: D15 (SEQ ID No. 41)
D36 5' gat gaa ttc tta atg gtg atg gtg atg atg ctc tcc ttg cac ctc cac 3' (SEQ ID No. 43)
- Name of plasmid:: pCXND3/htIL-20Rβ-His
- Template:: pCXND3/hIL-20Rβ
- Encoded Protein:: human IL-20Rβ, membrane-bound type, deletion of intracellular region, C-terminal His tag added
- Primers:: D15 (SEQ ID No. 41)
D17 5' gat gaa ttc tta atg gtg atg gtg atg atg cag ccg gcc cat ttt cca 3' (SEQ ID No. 44)
- Name of plasmid:: pCXND3/hIL-20Rα
- Template:: normal human lung-derived cDNA pool
- Encoded Protein:: human IL-20Rα, entire ORF, no tags
- Primers:: D22 5' gat gaa ttc ccc agc ggg cgt ggg act gag cag 3' (SEQ ID No. 45) D23 5' gat gaa ttc agg aaa caa aag gca aaa gga agt 3' (SEQ ID No. 46)
- Name of plasmid:: pCXND3/hEL-22R
- Template:: normal human small intestine-derived cDNA pool
- Encoded Protein:: human IL-22R, entire ORF, no tags
- Primers:: D37 5' gat gaa ttc ctc tgt gcc agc ccc gat gag gac 3' (SEQ ID No. 47) D39 5' gat gaa ttc cca agc ctt tcc cat tcc cct cag 3' (SEQ ID No. 48)
- Name of plasmid:: pCXND3/hIL.-20
- Template:: normal human lung-derived cDNA pool
- Encoded Protein:: human IL-20, entire ORF, no tags
- Primers:: D32 5' gat gaa ttc gct cct gtg gtc tcc aga ttt cag 3' (SEQ ID No. 49) D34 5' gat gaa ttc ata ttc tta gca gca gca tca ctt 3' (SEQ ID No. 50)
- Name of plasmid:: pCXND3/hIL-20-FLAG
- Template:: pCXND3/hIL-20
- Encoded Protein:: human IL-20, entire ORF, C-terminal FLAG tag added
- Primers:: D53 5' gat gaa ttc tcc acc atg aaa gcc tct agt ctt 3' (SEQ ID No. 51) D54

5' gat gaa ttc tta ctt gtc atc gtc gtc ctt gta gtc ttc tgt ctc ctc cat cca 3' (SEQ ID No. 52)

### <Composition of PCR reaction solution>

| | |
|---|---|
| KOD-Plus (TOYOBO) | 1 µL |
| 10 x PCR buffer (TOYOBO) | 5 µL |
| 2 mM dNTPs (TOYOBO) | 5 µL |
| 25 mM MgSO₄ (TOYOB | 2 µL |
| 20 µM primers | 1 µL each |
| template DNA | appropriate amount |

Water was added such that the total amount became 50 µL. Otherwise,

| | |
|---|---|
| TaKaRa ExTaq (TaKaRa) | 0.5 µL |
| TaqStart™ Antibody (CLONTECH) | 0.5 µL |
| 10 x ExTaq buffer (TaKaRa) | 5 µL |
| 2.5 mM dNTPs (TaKaRa) | 4 µL |
| 20 µM primers | 1 µL each |
| template DNA | appropriate amount |

Water was added such that the total amount became 50 µL.

### <PCR reaction conditions>

94°C, 2 minutes → 25 to 40 cycles x (94°C, 15 seconds; 60°C, 30 seconds; and 68°C, 2 minutes) → 72°C, 10 minutes, or
94°C, 2 minutes → 25 to 40 cycles x (94°C, 30 seconds; 60°C, 30 seconds; and 72°C, 2 minutes) → 72°C, 10 minutes

### [Example 8] Preparation of cells in which IL-20Rβ-associated molecules are expressed

The expression plasmid DNA prepared as above was digested with restriction enzyme *Pvu*I for linearization. Thereafter, it was introduced into CHO cells by electroporation using GENE-PULSER II (BIO-RAD). Conditions for the electroporation were as follows:
Cuvette: 0.4 cm electrode
Pulse: 1.5 kV, 25 µF

After completion of the electroporation, the cells were cultured in the presence of G418, so as to select cells into which the gene was introduced. The expressed protein was detected by Western analysis or the like using antibodies to tags.

Thus, the following cell lines were prepared:

### • Cell line 1

Protein to be expressed: human IL-20Rβ, only extracellular region, C-terminal FLAG tag added
Parent cells: CHO cells
Plasmid introduced: pCXND3/hsIL-20Rβ-FLAG

### • Cell line 2

Protein to be expressed: human IL-20Rβ, only extracellular region, C-terminal His tag added
Parent cells: CHO cells
Plasmid introduced: pCXND3/hsIL-20Rβ-His

### • Cell line 3

Protein to be expressed: human IL-20Rβ, membrane-bound type, deletion of intracellular region, C-terminal His tag added
Parent cells: CHO cells
Plasmid introduced: pCXND3/htIL-20Rβ-His

### • Cell line 4

Protein to be expressed: human IL-20, entire ORF, C-terminal FLAG tag added
Parent cells: CHO cells
Plasmid introduced: pCXND3/hIL-20-FLAG

In addition, a cell line growing depending on IL-20 (cell line 5) was prepared as follows. Specifically, pCXND3/hIL-20Rβ and pCXND3/hTL-22R were digested with *Pvu*I for linearization. Thereafter, they were introduced into Ba/F3 cells by electroporation using GENE-PULSER II (BIO-RAD). Conditions for the electroporation were as follows:
Cuvette: 0.4 cm electrode
Pulse: 0.33 kV, 950 µF
After completion of the electroporation, the cells were cultured in the presence of G418 and IL-20, so as to select cells growing depending on IL-20. Thus, cell line 5 was established.

### [Example 9] Preparation of IL-20Rβ-associated recombinant protein

### • Soluble human IL-20Rβ-FLAG protein

Cell line 1 was cultured in large quantity, and the culture supernatant was recovered. The culture supernatant was applied to a column filled with Q-Sepharose Fast Flow (Amersham Biosciences), which was an anion exchange resin. The column was washed with a washing buffer (50 mM sodium phosphate buffer, 0.01% Tween 20, pH 7.6), and an elution buffer (50 mM sodium phosphate buffer, 500 mM NaCl, 0.01% Tween 20, pH 7.2) was then applied thereto, so as to obtain a crude purification fraction containing a soluble human IL-20Rβ-FLAG protein. Subsequently, the crude purification fraction was subjected to the below-mentioned affinity purification using FLAG M2 (Freezer safe)-Agarose (SIGMA-ALDRICH). Specifically, the above crude purification fraction was applied to the above affinity resin-filled column, and the column was then washed with Tris-buffered saline (TaKaRa) containing 0.01% Tween 20, followed by elution with 100 mM glycine-HCl and 0.01% Tween 20, pH 3.5. 1M Tris-HCl, pH 8.0 was appropriately added to the eluted fraction for neutralization. Thereafter, the buffer was substituted by D-PBS(-) containing 0.01% Tween 20, using a PD-10 column (Amersham Biosciences). The resultant product was concentrated to an appropriate amount using Centriprep YM-10 (Millipore), and the thus obtained product was defined as a final purification product.

The purity of the final purification product was evaluated by SDS-PAGE, and the protein concentration was calculated relative to bovine gamma-globulin, using Dc Protein Assay (BIO-RAD).

### • Soluble human IL-20Rβ-His protein

Cell line 2 was cultured in large quantity, and the culture supernatant was recovered. 1/10 amount of 500 mM NaH₂PO₄ and 1.5M NaCl, pH 8.0 were added to the culture supernatant. The obtained mixture was applied to a column filled with Ni-NTA Superflow (QIAGEN), which was a carrier for metal chelate affinity chromatography. The column was then washed with a washing buffer (50 mM NaH₂PO₄, 300 mM NaCl, 5 mM imidazole, 0.01% Tween 20, pH 8.0). Thereafter, an elution buffer (50 mM NaH₂PO₄, 300 mM NaCl, 250 mM imidazole, 0.01% Tween 20, pH 8.0) was applied to the column, so as to obtain an affinity purification fraction containing a soluble human IL-20Rβ-His protein. Thereafter, the affinity purification fraction was concentrated to an appropriate amount using CentriprepYM-10 (Millipore), and the obtained concentrate was then subjected to gel filtration chromatography using HiLoad 16/60 Superdex 75 pg (Amersham Biosciences). A soluble human IL-20Rβ-His fraction was separated from impurities by this gel filtration chromatography. The obtained soluble human IL-20Rβ-His fraction was concentrated to an appropriate amount using CentriprepYM-10 again, and the thus obtained product was defined as a final purification product. It is to be noted that D-PBS(-) 0.01% Tween 20 was used as a buffer for gel filtration chromatography.

The purity of the final purification product was evaluated by SDS-PAGE, and the protein concentration was calculated relative to bovine γ-globulin, using Dc Protein Assay (BIO-RAD).

### • Human IL-20-FLAG protein

Cell line 4 was cultured in large quantity, and the culture supernatant was recovered. The culture supernatant was applied to a column filled with FLAG M2 (Freezer safe)-Agarose (SIGMA-ALDRICH) affinity resin, and the column was then washed with Tris-buffered saline (TaKaRa), followed by elution with 100 mM glycine-HCl, pH 3.5. Thereafter, 1M Tris-HCl, pH 8.0 was appropriately added to the eluted fraction for neutralization. Thereafter, the buffer was substituted by D-PBS(-), using a PD-10 column (Amersham Biosciences). The resultant product was concentrated to an appropriate amount using CentriprepYM-10 (Millipore), and the thus obtained product was defined as a final purification product.

The purity of the final purification product was evaluated by SDS-PAGE, and the protein concentration was calculated relative to bovine γ-globulin, using Dc Protein Assay (BIO-RAD).

### [Example 10] Preparation of anti-IL-20Rβ antibody

### Preparation of anti-IL-20Rβ rabbit polyclonal antibody

Based on the amino acid sequence (SEQ ID No. 16) of human IL-20Rβ, the following peptide was synthesized (Sawady Technology Co., Ltd.).

NP2 : NH2 - CDVTDDITATVPYNLRVRAT – COOH (SEQ ID No. 53)

NP3 : NH2 - CYWRREPGAEEHVKMVRSGG – COOH (SEQ ID No. 54)

By a method using m-maleimidebenzoyl-N-hydroxysuccinimide ester (MBS), each peptide was bound to keyhole limpet hemocyanin (KLH) via cysteine at the amino terminus. A rabbit (Japanese white rabbit, female) was immunized with the obtained peptide several times, so as to obtain antiserum. Affinity purification was carried out using a column on which a peptide was immobilized, so as to prepare a monospecific antibody specifically reacting with a peptide. The titer of the obtained antibody was analyzed by ELISA method using, as an antigen, the peptide used in the immunization, or by FACS analysis using the above-described human IL-20Rβ expressing cells (cell line 3) (Figure 10).

### Preparation of anti-IL-20Rβ mouse monoclonal antibody

A mouse was immunized with the soluble human IL-20Rβ-FLAG protein prepared as above. For the initial immunization, the protein solution was mixed with a Freund's complete adjuvant (FCA) for emulsification, and 100 µg of the protein per mouse was administered into several sites of the abdominal subcutis. For the subsequent immunizations, the protein solution was mixed with a Freund's incomplete adjuvant (FIA) for emulsification, and 50 µg of the protein per mouse was administered into several sites of the abdominal subcutis. The blood was collected from three immunized mice, and the serum was separated therefrom. Using the serum, an increase in the antibody titer was assayed by the ELISA method (Figure 11).

ELISA was carried out based on a general technique of operation, using a soluble human IL-20Rβ-His protein as an antigen. Splenic cells were prepared from mice in which an increase in the antibody titer was observed. Thereafter, cell fusion was carried out between the splenic cells and P3U1 cells as mouse myeloma cells by a general method using polyethylene glycol. Thereafter, the fused cells were inoculated on a 96-well plate. This was then cultured in a medium containing HAT (hypoxanthine, aminopterine, and thymidine), so as to select only hybridomas. The culture supernatant of the hybridomas was subjected to ELISA using a soluble human IL-20Rβ-His protein as an antigen, so as to select wells wherein hybridomas producing an antibody to IL-20Rβ existed.

If the culture supernatant of the hybridomas is screened by a growth assay using IL-20-dependent cell line (cell line 5) at the same time, hybridomas producing an antibody having neutralizing activity can also be selected. The growth assay using cell line 5 can be carried out as follows. This is to say, cell line 5 is washed with a medium containing no cytokines, and an appropriate number of cells (2,000 to 50,000 cells/well, and generally 20,000 cells/well) are inoculated onto a 96-well plate. Thereafter, an antibody sample and cytokines (human IL-20-FLAG protein) are appropriately added thereto, and the mixture is cultured at 37°C for an appropriate time (16 to 96 hours, and generally 24 hours). After completion of the culture, the number of cells is measured by a general method such as an MTT assay or WST-8 assay, so as to assay the growth level of the cells. WST-8 assay can be carried out by adding an appropriate amount (generally an amount of 1/10 of the culture solution) of Cell Count Reagent SF (Nacalai Tesque, Inc.) to the culture solution, culturing the mixture for an appropriate time (generally 2 hours), and measuring an absorbance at 450 nm.

Wells in which binding activity was observed were subjected to limiting dilution to carry out single cloning of hybridomas, and the binding activity of an antibody was assayed by the ELISA method again, so as to isolate hybridoma clones (MK8, MK10, MK14, MK23, MK24, MK27, and MK28) producing antibodies binding to IL-20Rβ (Figure 12).

### Industrial Applicability

The pharmaceutical composition and diagnostic agent of the present invention specifically bind to IL-20Rβ, which is expressed in lung carcinoma cells more significantly than in normal cells. Accordingly, the pharmaceutical composition and diagnostic agent of the present invention are useful for treatment and diagnosis of lung carcinoma. The present invention can provide a therapeutic method and a diagnostic method, which are extremely effective for lung carcinoma including non small cell lung carcinoma whose recovery rate is extremely low by the exiting therapeutic methods.

All publications, patents and patent applications cited herein are incorporated herein by reference in their entirety.

## Claims

1. A pharmaceutical composition for treating lung carcinoma, which comprises, as an active ingredient, an antibody binding to the β-chain of an interleukin-20 receptor.

2. The pharmaceutical composition according to claim 1, wherein the antibody is a monoclonal antibody or polyclonal antibody.

3. The pharmaceutical composition according to claim 1 or 2, wherein the antibody is a human antibody, humanized antibody, or chimeric antibody.

4. A pharmaceutical composition for treating lung carcinoma comprising, an active ingredient, a polynucleotide which has a nucleotide sequence complementary to the nucleotide sequence of a polynucleotide encoding the β-chain of an interleukin-20 receptor and which is an antisense polynucleotide capable of suppressing the expression of the polynucleotide.

5. A diagnostic agent for lung carcinoma, which comprises an antibody capable of binding to the β-chain of an interleukin-20 receptor.

6. The diagnostic agent according to claim 5, wherein the antibody is a monoclonal antibody or polyclonal antibody.

7. The diagnostic agent according to claim 5 or 6, wherein the antibody is a human antibody, humanized antibody, or chimeric antibody.

8. A method for detecting lung carcinoma in mammals using the diagnostic agent according to any one of claims 5 to 7.

9. A method for detecting lung carcinoma, which comprises detecting the expression of the β-chain of an interleukin-20 receptor in a sample obtained from a mammal suspected to have lung carcinoma.

10. The method according to claim 9, wherein an antibody capable of binding to the β-chain of an interleukin-20 receptor is used to detect the expression.

11. The method according to claim 9, wherein the PCR method is used to detect the expression.


SEQUENCE LISTING

<110> Chugai Seiyaku Kabushiki Kaisha

<120> A Therapeutic Reagent for Lung Carcinoma

<130> PH-1741-PCT

<150> JP 2002-124743
<151> 2002-04-25

<160> 54

<170> PatentIn Ver. 2.1

<210> 1
<211> 44
<212> DNA
<213> Artificial Sequence

<220>
<223> Description of Artificial Sequence: Synthetic DNA

<400> 1
ctaatacgac tcactatagg gctcgagcgg ccgcccgggc aggt 44

<210> 2
<211> 10
<212> DNA

<213> Artificial Sequence

<220>
<223> Description of Artificial Sequence: Synthetic DNA

<400> 2
acctgcccgg                                                                10

<210> 3
<211> 43
<212> DNA
<213> Artificial Sequence

<220>
<223> Description of Artificial Sequence: Synthetic DNA

<400> 3
tgtagcgtga agacgacaga aagggcgtgg tgcggagggc ggt                           43

<210> 4
<211> 11
<212> DNA
<213> Artificial Sequence

<220>
<223> Description of Artificial Sequence: Synthetic DNA

<400> 4
accgccctcc g                                                              11

<210> 5

<211> 22

<212> DNA

<213> Artificial Sequence

<220>

<223> Description of Artificial Sequence: Synthetic DNA

<400> 5

ctaatacgac tcactatagg gc                                                22

<210> 6

<211> 21

<212> DNA

<213> Artificial Sequence

<220>

<223> Description of Artificial Sequence: Synthetic DNA

<400> 6

tgtagcgtga agacgacaga a                                                 21

<210> 7

<211> 22

<212> DNA

<213> Artificial Sequence

<220>

<223> Description of Artificial Sequence: Synthetic DNA

<400> 7
tcgagcggcc gcccgggcag gt                                                22

<210> 8
<211> 22
<212> DNA
<213> Artificial Sequence

<220>
<223> Description of Artificial Sequence: Synthetic DNA

<400> 8
agggcgtggt gcggagggcg gt                                                22

<210> 9
<211> 22
<212> DNA
<213> Artificial Sequence

<220>
<223> Description of Artificial Sequence: Synthetic DNA

<400> 9
ccagggtttt cccagtcacg ac                                                22

<210> 10
<211> 22

<212> DNA
<213> Artificial Sequence

<220>
<223> Description of Artificial Sequence: Synthetic DNA

<400> 10
tcacacagga aacagctatg ac                                                22

<210> 11
<211> 24
<212> DNA
<213> Artificial Sequence

<220>
<223> Description of Artificial Sequence: Synthetic DNA

<400> 11
ccacattggg ctcacagacc tcag                                              24

<210> 12
<211> 24
<212> DNA
<213> Artificial Sequence

<220>
<223> Description of Artificial Sequence: Synthetic DNA

<400> 12

gtaggccaca aggaactcaa actg                                                24

<210> 13
<211> 28
<212> DNA
<213> Artificial Sequence

<220>
<223> Description of Artificial Sequence: Synthetic DNA

<400> 13
gatgagaagg acccacggcg tctgttcg                                            28

<210> 14
<211> 28
<212> DNA
<213> Artificial Sequence

<220>
<223> Description of Artificial Sequence: Synthetic DNA

<400> 14
gagacaatcc agcagcccag gagggaca                                            28

<210> 15
<211> 2056
<212> DNA
<213> Homo sapiens

<220>

<221> CDS

<222> (250).. (1185)

<400> 15

aaagttacat tttctctgga actctcctag gccactccct gctgatgcaa catctgggtt 60

tgggcagaaa ggagggtgct tcggagcccg ccctttctga gcttcctggg ccggctctag 120

aacaattcag gcttcgctgc gactcagacc tcagctccaa catatgcatt ctgaagaaag 180

atggctgaga tggacagaat gctttatttt ggaaagaaac aatgttctag gtcaaactga 240

gtctaccaaa tgcagacttt cacaatggtt ctagaagaaa tctggacaag tcttttcatg 300

tggtttttct acgcattgat tccatgtttg ctcacagatg aagtggccat tctgcctgcc 360

cctcagaacc tctctgtact ctcaaccaac atgaagcatc tcttgatgtg gagcccagtg 420

atcgcgcctg gagaaacagt gtactattct gtcgaatacc agggggagta cgagagcctg 480

tacacgagcc acatctggat ccccagcagc tggtgctcac tcactgaagg tcctgagtgt 540

gatgtcactg atgacatcac ggccactgtg ccatacaacc ttcgtgtcag ggccacattg 600

ggctcacaga cctcagcctg gagcatcctg aagcatccct ttaatagaaa ctcaaccatc 660

cttacccgac ctgggatgga gatcaccaaa gatggcttcc acctggttat tgagctggag 720

gacctggggc cccagtttga gttccttgtg gcctactgga ggagggagcc tggtgccgag 780

gaacatgtca aaatggtgag gagtgggggt attccagtgc acctagaaac catggagcca 840

ggggctgcat actgtgtgaa ggcccagaca ttcgtgaagg ccattgggag gtacagcgcc 900

ttcagccaga cagaatgtgt ggaggtgcaa ggagaggcca ttcccctggt actggccctg 960

tttgcctttg ttggcttcat gctgatcctt gtggtcgtgc cactgttcgt ctggaaaatg 1020

ggccggctgc tccagtactc ctgttgcccc gtggtggtcc tcccagacac cttgaaaata 1080

accaattcac cccagaagtt aatcagctgc agaagggagg aggtggatgc ctgtgccacg 1140

gctgtgatgt ctcctgagga actcctcagg gcctggatct cataggtttg cggaagggcc 1200

caggtgaagc cgagaacctg gtctgcatga catggaaacc atgaggggac aagttgtgtt 1260

tctgttttcc gccacggaca agggatgaga gaagtaggaa gagcctgttg tctacaagtc 1320

tagaagcaac catcagaggc agggtggttt gtctaacaga acactgactg aggcttaggg 1380

gatgtgacct ctagactggg ggctgccact tgctggctga gcaaccctgg gaaaagtgac 1440

ttcatccctt cggtcctaag ttttctcatc tgtaatgggg gaattaccta cacacctgct 1500
aaacacacac acacagagtc tctctctata tatacacacg tacacataaa tacacccagc 1560
acttgcaagg ctagagggaa actggtgaca ctctacagtc tgactgattc agtgtttctg 1620
gagagcagga cataaatgta tgatgagaat gatcaaggac tctacacact gggtggcttg 1680
gagagcccac tttcccagaa taatccttga gagaaaagga atcatgggag caatggtgtt 1740
gagttcactt caagcccaat gccggtgcag aggggaatgg cttagcgagc tctacagtag 1800
gtgacctgga ggaaggtcac agccacactg aaaatgggat gtgcatgaac acggaggatc 1860
catgaactac tgtaaagtgt tgacagtgtg tgcacactgc agacagcagg tgaaatgtat 1920
gtgtgcaatg cgacgagaat gcagaagtca gtaacatgtg catgtttgtt gtgctccttt 1980
tttctgttgg taaagtacag aattcagcaa ataaaaaggg ccaccctggc caaaagcggt 2040
aaaaaaaaaa aaaaaa 2056

<210> 16
<211> 311
<212> PRT
<213> Homo sapiens

<400> 16
Met Gln Thr Phe Thr Met Val Leu Glu Glu Ile Trp Thr Ser Leu Phe
1               5                   10                  15

Met Trp Phe Phe Tyr Ala Leu Ile Pro Cys Leu Leu Thr Asp Glu Val
            20                  25                  30

Ala Ile Leu Pro Ala Pro Gln Asn Leu Ser Val Leu Ser Thr Asn Met
        35                  40                  45

Lys His Leu Leu Met Trp Ser Pro Val Ile Ala Pro Gly Glu Thr Val
    50                  55                  60

Tyr Tyr Ser Val Glu Tyr Gln Gly Glu Tyr Glu Ser Leu Tyr Thr Ser
65                  70                  75                  80

His Ile Trp Ile Pro Ser Ser Trp Cys Ser Leu Thr Glu Gly Pro Glu
                85                  90                  95

Cys Asp Val Thr Asp Asp Ile Thr Ala Thr Val Pro Tyr Asn Leu Arg
            100                 105                 110

Val Arg Ala Thr Leu Gly Ser Gln Thr Ser Ala Trp Ser Ile Leu Lys
        115                 120                 125

His Pro Phe Asn Arg Asn Ser Thr Ile Leu Thr Arg Pro Gly Met Glu
    130                 135                 140

Ile Thr Lys Asp Gly Phe His Leu Val Ile Glu Leu Glu Asp Leu Gly
145                 150                 155                 160

Pro Gln Phe Glu Phe Leu Val Ala Tyr Trp Arg Arg Glu Pro Gly Ala
                165                 170                 175

Glu Glu His Val Lys Met Val Arg Ser Gly Gly Ile Pro Val His Leu
            180                 185                 190

Glu Thr Met Glu Pro Gly Ala Ala Tyr Cys Val Lys Ala Gln Thr Phe
        195                 200                 205

Val Lys Ala Ile Gly Arg Tyr Ser Ala Phe Ser Gln Thr Glu Cys Val

    210                 215                 220

Glu Val Gln Gly Glu Ala Ile Pro Leu Val Leu Ala Leu Phe Ala Phe
225                 230                 235                 240

Val Gly Phe Met Leu Ile Leu Val Val Val Pro Leu Phe Val Trp Lys
                245                 250                 255

Met Gly Arg Leu Leu Gln Tyr Ser Cys Cys Pro Val Val Val Leu Pro
            260                 265                 270

Asp Thr Leu Lys Ile Thr Asn Ser Pro Gln Lys Leu Ile Ser Cys Arg
        275                 280                 285

Arg Glu Glu Val Asp Ala Cys Ala Thr Ala Val Met Ser Pro Glu Glu
    290                 295                 300

Leu Leu Arg Ala Trp Ile Ser
305                 310

<210> 17
<211> 24
<212> DNA
<213> Artificial Sequence

<220>
<223> Description of Artificial Sequence: Synthetic DNA

<400> 17

caggaggaag aggaggtgaa acat                                                24

<210> 18

<211> 24

<212> DNA

<213> Artificial Sequence

<220>

<223> Description of Artificial Sequence: Synthetic DNA

<400> 18

tcttggagct gaggggtgta tgag                                                24

<210> 19

<211> 24

<212> DNA

<213> Artificial Sequence

<220>

<223> Description of Artificial Sequence: Synthetic DNA

<400> 19

tccatgggct tcctcgtcgc agta                                                24

<210> 20

<211> 24

<212> DNA

<213> Artificial Sequence

<220>
<223> Description of Artificial Sequence: Synthetic DNA

<400> 20
gggcagcgtt tggggcatag gaca                                                24

<210> 21
<211> 24
<212> DNA
<213> Artificial Sequence

<220>
<223> Description of Artificial Sequence: Synthetic DNA

<400> 21
atcaggagcc aaaccccaaa atct                                                24

<210> 22
<211> 24
<212> DNA
<213> Artificial Sequence

<220>
<223> Description of Artificial Sequence: Synthetic DNA

<400> 22
cttccccatc tccttcccct tcaa                                                24

<210> 23

<211> 24

<212> DNA

<213> Artificial Sequence

<220>

<223> Description of Artificial Sequence: Synthetic DNA

<400> 23

gtgcaagcca aagatggaaa catt                                                24

<210> 24

<211> 24

<212> DNA

<213> Artificial Sequence

<220>

<223> Description of Artificial Sequence: Synthetic DNA

<400> 24

tattctgtct cctccatcca ttgc                                                24

<210> 25

<211> 24

<212> DNA

<213> Artificial Sequence

<220>

<223> Description of Artificial Sequence: Synthetic DNA

<400> 25
tattctgtct cctccatcca ttgc                                                24

<210> 26
<211> 24
<212> DNA
<213> Artificial Sequence

<220>
<223> Description of Artificial Sequence: Synthetic DNA

<400> 26
tattctgtct cctccatcca ttgc                                                24

<210> 27
<211> 24
<212> DNA
<213> Artificial Sequence

<220>
<223> Description of Artificial Sequence: Synthetic DNA

<400> 27
tatgggcaaa agaaatggct gaat                                                24

<210> 28
<211> 24
<212> DNA
<213> Artificial Sequence

<220>
<223> Description of Artificial Sequence: Synthetic DNA

<400> 28
gggaccacgt tctgtttgat ttag                                              24

<210> 29
<211> 22
<212> DNA
<213> Artificial Sequence

<220>
<223> Description of Artificial Sequence: Synthetic DNA

<400> 29
ccaacatgaa gcatctcttg at                                                22

<210> 30
<211> 22
<212> DNA
<213> Artificial Sequence

<220>
<223> Description of Artificial Sequence: Synthetic DNA

<400> 30
cccccactcc tcaccatttt ga                                                22

<210> 31
<211> 24
<212> DNA
<213> Artificial Sequence

<220>
<223> Description of Artificial Sequence: Synthetic DNA

<400> 31
ttcatcctac ccccacgcca atcc                                                24

<210> 32
<211> 24
<212> DNA
<213> Artificial Sequence

<220>
<223> Description of Artificial Sequence: Synthetic DNA

<400> 32
aggtgcaggc ggcttggtga cata                                                24

<210> 33
<211> 24
<212> DNA
<213> Artificial Sequence

<220>
<223> Description of Artificial Sequence: Synthetic DNA

<400> 33
aagccaccaa tgccaccaga gtca 24

<210> 34
<211> 24
<212> DNA
<213> Artificial Sequence

<220>
<223> Description of Artificial Sequence: Synthetic DNA

<400> 34
caggcccttt cagcagatag agtt 24

<210> 35
<211> 22
<212> DNA
<213> Artificial Sequence

<220>
<223> Description of Artificial Sequence: Synthetic DNA

<400> 35
agtgtgcaag ccaaagatgg aa 22

<210> 36
<211> 22
<212> DNA

<213> Artificial Sequence

<220>
<223> Description of Artificial Sequence: Synthetic DNA

<400> 36
ctggctgtat ttcttcattg ct                                                22

<210> 37
<211> 24
<212> DNA
<213> Artificial Sequence

<220>
<223> Description of Artificial Sequence: Synthetic DNA

<400> 37
gatgctgaga gctgttacct tgtc                                              24

<210> 38
<211> 24
<212> DNA
<213> Artificial Sequence

<220>
<223> Description of Artificial Sequence: Synthetic DNA

<400> 38
aaatgacaca gggaacaaac cagt                                              24

<210> 39

<211> 33

<212> DNA

<213> Artificial Sequence

<220>

<223> Description of Artificial Sequence: Synthetic DNA

<400> 39

gatgaattcg aagaaagatg gctgagatgg aca 33

<210> 40

<211> 33

<212> DNA

<213> Artificial Sequence

<220>

<223> Description of Artificial Sequence: Synthetic DNA

<400> 40

gatgaattct ggcggaaaac agaaacacaa ctt 33

<210> 41

<211> 33

<212> DNA

<213> Artificial Sequence

<220>

<223> Description of Artificial Sequence: Synthetic DNA

<400> 41
gatgaattct ccaccatgca gactttcaca atg                                33

<210> 42
<211> 54
<212> DNA
<213> Artificial Sequence

<220>
<223> Description of Artificial Sequence: Synthetic DNA

<400> 42
gatgaattct tacttgtcat cgtcgtcctt gtagtcctct ccttgcacct ccac        54

<210> 43
<211> 48
<212> DNA
<213> Artificial Sequence

<220>
<223> Description of Artificial Sequence: Synthetic DNA

<400> 43
gatgaattct taatggtgat ggtgatgatg ctctccttgc acctccac              48

<210> 44
<211> 48

<212> DNA

<213> Artificial Sequence

<220>

<223> Description of Artificial Sequence: Synthetic DNA

<400> 44

gatgaattct taatggtgat ggtgatgatg cagccggccc attttcca 48

<210> 45

<211> 33

<212> DNA

<213> Artificial Sequence

<220>

<223> Description of Artificial Sequence: Synthetic DNA

<400> 45

gatgaattcc ccagcgggcg tgggactgag cag 33

<210> 46

<211> 33

<212> DNA

<213> Artificial Sequence

<220>

<223> Description of Artificial Sequence: Synthetic DNA

<400> 46

gatgaattca ggaaacaaaa ggcaaaagga agt                                    33

<210> 47
<211> 33
<212> DNA
<213> Artificial Sequence

<220>
<223> Description of Artificial Sequence: Synthetic DNA

<400> 47
gatgaattcc tctgtgccag ccccgatgag gac                                    33

<210> 48
<211> 33
<212> DNA
<213> Artificial Sequence

<220>
<223> Description of Artificial Sequence: Synthetic DNA

<400> 48
gatgaattcc caagcctttc ccattcccct cag                                    33

<210> 49
<211> 33
<212> DNA
<213> Artificial Sequence

<220>

<223> Description of Artificial Sequence: Synthetic DNA

<400> 49

gatgaattcg ctcctgtggt ctccagattt cag 33

<210> 50

<211> 33

<212> DNA

<213> Artificial Sequence

<220>

<223> Description of Artificial Sequence: Synthetic DNA

<400> 50

gatgaattca tattcttagc agcagcatca ctt 33

<210> 51

<211> 33

<212> DNA

<213> Artificial Sequence

<220>

<223> Description of Artificial Sequence: Synthetic DNA

<400> 51

gatgaattct ccaccatgaa agcctctagt ctt 33

<210> 52

<211> 54

<212> DNA

<213> Artificial Sequence

<220>

<223> Description of Artificial Sequence: Synthetic DNA

<400> 52

gatgaattct tacttgtcat cgtcgtcctt gtagtcttct gtctcctcca tcca 54

<210> 53

<211> 20

<212> PRT

<213> Artificial Sequence

<220>

<223> Description of Artificial Sequence: Synthetic peptide

<400> 53

Cys Asp Val Thr Asp Asp Ile Thr Ala Thr Val Pro Tyr Asn Leu Arg

1               5                   10                  15

Val Arg Ala Thr

            20

<210> 54

<211> 20

<212> PRT

<213> Artificial Sequence

<220>
<223> Description of Artificial Sequence: Synthetic peptide

<400> 54
Cys Tyr Trp Arg Arg Glu Pro Gly Ala Glu Glu His Val Lys Met Val
1               5                   10                  15

Arg Ser Gly Gly
            20